(19) Europäisches Patentamt
European Patent Office
Office européen des brevets

(11) Publication number: 0 444 889 A1

# (12) EUROPEAN PATENT APPLICATION

(21) Application number: 91301557.4

(51) Int. Cl.[5]: C07D 477/00

(22) Date of filing: 26.02.91

(30) Priority: 26.02.90 US 485096
20.07.90 US 556591
04.02.91 US 650111

(43) Date of publication of application:
04.09.91 Bulletin 91/36

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Rano, Thomas A.
175 Hana Road
Edison, NJ 08817 (US)
Inventor: Greenlee, Mark L.
1470 Campbell Street
Rahway, NJ 07065 (JP)
Inventor: Dininno, Frank P.
5 Benjamin Court
Old Bridge, NJ 08857 (US)

(74) Representative: Thompson, John Dr. et al
Merck & Co., Inc. European Patent
Department Terlings Park Eastwick Road
Harlow, Essex CM20 2QR (GB)

## (54) Novel synthesis of carbapenem intermediates.

(57) Carbapenems having the formula:

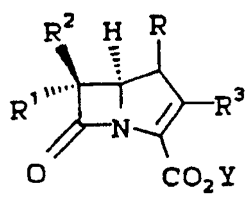

I

are prepared by a novel reaction of a readily accessible carbapenem intermediate and an organostannane. The reaction, which is mediated by a palladium compound and, optionally, a phosphine ligand, proceeds at remarkably mild temperatures in reproducibly good yields.

EP 0 444 889 A1

# NOVEL SYNTHESIS OF CARBAPENEM INTERMEDIATES

## BACKGROUND OF THE INVENTION

The present invention relates to a novel synthesis of key intermediates employed in the synthesis of antibacterial agents of the C-2-substituted carbapenem class. The synthesis utilizes the palladium mediated coupling reaction between an activated carbapenem intermediate and a suitably substituted organostannane.

Thienamycin was an early carbapenem antibacterial agent having a broad spectrum; it has the following formula:

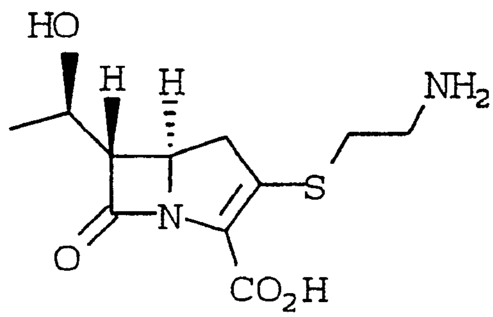

Later, N-formimidoyl thienamycin was discovered; it has the formula:

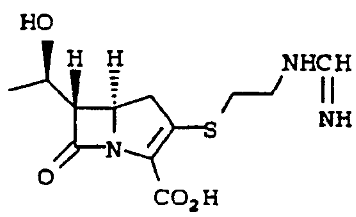

More recently, carbapenem antibacterial agents have been described which have a 2-substituent which is an organo moiety, optionally substituted. These agents are described in U.S. Pat. Nos. 4,260,627, 4,543,257 and 4,775,669, all assigned to Merck & Co.,Inc. and incorporated herein by reference, and have the formula:

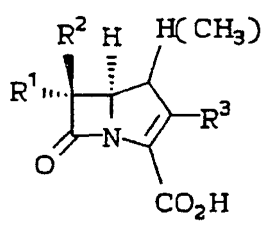

Previous syntheses of this class of carbapenem antibiotics, disclosed by L. Cama and B. G. Christensen, Tetrahedron Lett., **21**, 2013(1980), L Cama et al, Tetrahedron, **39**, 2531(1983), R. Guthikonda et al., J. Med. Chem., **30**, 871(1987) and S. Schmitt et al., J. Antibiot., **41**, 780(1988) and disclosed in U.S. Pat. Appl. SN163,240, now abandoned, U.S. Pat. Appl. SN171,244, now abandoned, U.S. Pat. No. 4,465,632, assigned to Merck & Co., Inc., were non-convergent and required extensive use of protecting groups and/or functional group equivalents due to the incompatibility of some functional groups to the chemical transformations employed. The above cited documents are all incorporated herein by reference.

Therefore it is an object of the present invention to provide a synthesis of the carbapenem intermediate of the formula I:

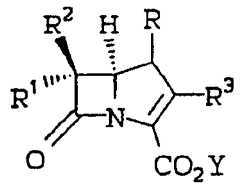

I

from the intermediate II of the formula:

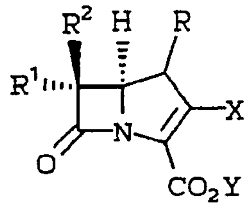

II

wherein the groups R, $R^1$, $R^2$, $R^3$, X and Y are defined hereinbelow.

It is an additional object of the present invention to provide a synthesis of the intermediate I of the formula illustrated hereinabove, wherein the reaction between intermediate II and a suitably substituted organostannane is mediated by a palladium compound and, optionally, a phosphine ligand.

It is a final object of the present invention to provide a synthesis of an intermediate of the Formula I which may be deprotected to provide a C-2-substituted carbapenem antibiotic.

## SUMMARY OF THE INVENTION

The present invention provides a novel PROCESS FOR PREPARING CARBAPENEM COMPOUNDS of the Formula I:

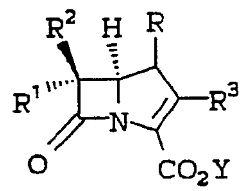

I

wherein:

R is H or $CH_3$;

$R^1$ and $R^2$ are independently H, $CH_3$-, $CH_3CH_2$-, $(CH_3)_2CH$-, $R^4OCH_2$-, $CH_3CH(OR^4)$-, $(CH_3)_2C(OR^4)$-, $FCH_2CH(OR^4)$-, $F_2CHCH(OR^4)$-, $F_3CCH(OR^4)$-, $CH_3CH(F)$-, $CH_3CF_2$-, or $(CH_3)_2C(F)$-;

wherein $R^4$ is hydrogen or $R^5$;

wherein $R^5$ is triorganosilyl, p-nitrobenzyloxycarbonyl or allyloxycarbonyl.

$R^3$ is a mono- or disubstituted or unsubstituted group which is: alkyl, alkenyl, alkynyl, phenyl, furyl, thienyl, dibenzofuranyl, phenanthrenyl, naphthyl, fluoren-9-onyl, pyridyl, phenylpyridyl, biphenyl, dibenzothienyl, 9-oxodibenzothienyl or 9,9-dioxodibenzothienyl;

wherein the substituent (or substituents) is:

a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;

b) a mono- or disubstituted or unsubstituted group which is: alkyl, alkenyl and alkynyl wherein the substituent (or substituents) is selected from a) hereinabove;

Y is allyl, substituted allyl, benzyl, substituted benzyl, alkyl, substituted alkyl or triorganosilyl;

COMPRISING THE STEP OF treating the carbapenem compound of the Formula II:

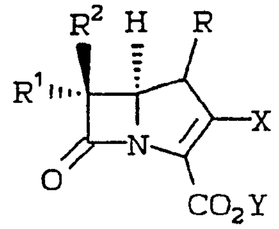

II

having the same substituents R, $R^1$, $R^2$, and Y as the compound of Formula I and

wherein X is trifluoromethanesulfonyloxy, methanesulfonyloxy, p-toluenesulfonyloxy, chloro, bromo, iodo or diphenylphosphonyloxy;

with 0.9 to 1.5 molar equivalents of an organostannane of the Formula III:

$$R^s{}_3Sn\text{-}R^3 \quad III$$

wherein $R^s$ is lower alkyl, and $R^3$ is same as described for Formula I;

IN THE PRESENCE OF 1 to 10 mole percent of a palladium compound and 0.5 to 3 molar equivalents of a halide source in an aprotic polar coordinating solvent and zero to 8.0 molar equivalents, relative to the palladium compound, of a phosphine of Formula IV:

$$P(R^p)_3 \quad IV$$

wherein $R^p$ is a group substituted from one to three times or unsubstituted which is: phenyl, 2-furyl or 2-thienyl;

wherein the substituent (or substituents) is lower alkyl or lower alkoxy.

As used herein, the term "alkyl" means carbon fragments having up to 20 carbon atoms and includes "lower alkyl". Examples of alkyl groups include octyl, nonyl, undecyl, dodecyl, tridecyl, tetradecyl, eicosyl and the like.

As used herein, the term "alkenyl" means those alkenyl groups of from 2 to 20 carbon atoms. Examples of alkenyl groups include vinyl, allyl, isopropenyl, pentenyl, hexenyl, 2-butenyl, 2-methyl-butenyl and the like.

As used herein, the term "alkynyl" means those alkynyl groups of from 2 to 20 carbon atoms. Examples of alkynyl groups include ethynyl, propargyl, 3-methyl-1-pentynyl, 2-heptynyl and the like.

As used herein, the term "lower alkyl" means those alkyl groups of from 1 to 7 carbon atoms. Examples of lower alkyl groups include methyl, ethyl, propyl, isopropyl, n-butyl, sec- and tert-butyl and the like.

As used herein, the term "lower alkoxy" means those alkoxy groups having an oxygen substituted with a lower alkyl. Examples of lower alkoxy include methoxy, ethoxy, propoxy, t-butoxy and the like.

As used herein in the definition of Y, the term "substituted benzyl" means those benzyl groups which serve as carboxyl protecting groups having a substituent (or substituents) familiar to those in the art. Such groups include those benzyl groups exemplified by 4-nitrobenzyl, 4-methoxybenzyl, 2-nitrobenzyl, benzhydryl and the like.

As used herein in the definition of Y, the term "substituted alkyl" means those alkyl groups which serve as carboxyl protecting groups having a substituent (or substituents) familiar to those in the art. Such groups include those alkyl groups exemplified by 2,2,2-trichloroethyl, trimethylsilylethyl, phenylsulfonylethyl and the like.

As used herein in the definition of Y, the term "substituted allyl" means those allyl groups which serve as carboxyl protecting groups having a substituent (or substituents) familiar to those in the art. Such groups include those allyl groups exemplified by methallyl, crotyl, dimethallyl, 2-chloroallyl, 2,3-dichloroallyl and the like.

As used herein in the definition of $R^5$ and Y, the term "triorganosilyl" means those silyl groups trisubstituted by lower alkyl groups or aryl groups or combinations thereof and wherein one substituent may be a lower alkoxy group. Examples of triorganosilyl groups include trimethylsilyl, triethylsilyl, t-butyldimethylsilyl, triphenylsilyl, dimethylphenylsilyl, phenyl-t-butylmethoxysilyl and the like.

As used herein, the term "palladium compound" means palladium(0) or palladium(II) compounds. Palladium(0) compounds are exemplified by $Pd(PPh_3)_4$, $Pd(DBA)_2$, $Pd_2(DBA)_3$·solvent and the like. Palladium(II) compounds are exemplified by $(CH_3CN)_2PdCl_2$, $Pd(OAc)_2$, $(Ph_3P)_2PdCl_2$, $(PhCN)_2PdCl_2$ and the like. The term "solvent" in the above examples of palladium(0) means solvents known in the art to be useful in the formation of such compounds and includes chloroform, benzene, toluene, methylene chloride and the like.

As used herein, the term "halide source" includes metal halides and substituted ammonium halides. The term "metal halide" includes lithium chloride, lithium bromide, sodium chloride, sodium bromide, potassium bromide, zinc bromide, zinc chloride and the like. The term "substituted ammonium halides" includes diisopropylamine hydrochloride, tetrabutylammonium chloride, triethylamine hydrochloride and the like.

As used herein, the term "aprotic polar coordinating solvent" includes tetrahydrofuran, 1,2-dimethoxyethane, dioxane, acetonitrile, acetone, hexamethylphosphoramide, dimethylformamide, 1,3-dimethyl-3,4,5,6-tetrahydro-2(1H)-pyrimidinone, 1,3-dimethyl-2-imidazolidinone (DMI), sulfolane, dimethylsulfoxide, 1-methyl-2-pyrrolidinone, dimethylacetamide and the like and mixtures of the above listed solvents.

With reference to the above definitions, "alkyl", "alkenyl" and "alkynyl" means a straight or branched chain aliphatic hydrocarbon radical.

It is understood that the term "substituted" in part "a)" of the substituent groups of $R^3$ only applies to those substituents which may be substituted (e.g., amino, alkoxy, carbonyl, carbamoyl, etc.).

It is intended that the definitions of any substituent (e.g., $R^s$, substituents on $R^3$, etc.) in a particular compound be independent of its definitions elsewhere in the compound. Thus $R^s{}_3SnR^3$ represents $Me_3SnR^3$, $Me_2EtSnR^3$, etc. It is also intended that $R^3$ and $R^s$ might be the same if $R^3$ is a lower alkyl.

If $R^3$ is an aryl or heteroaryl group (eg. phenyl, thienyl, furyl etc.), it is intended that the point of attachment, on $R^3$, to the carbapenem nucleus as defined in Formula I may be at any of the carbon atoms of $R^3$ not occupied by a substituent or part of a ring fusion.

It is understood that if any substituent functional group which is part of $R^3$ (e.g., amino) is incompatible with the chemical transformations of the present invention the group may be selectively protected, by techniques known in the art, prior to preparation of the stannane III. Such protection may then be removed from $R^3$, by techniques known in the art, after $R^3$ is incorporated into the carbapenem intermediate I.

It is understood that substitution on a pyridyl or phenylpyridyl group representing $R^3$ may occur at the nitrogen atom of the pyridine ring, thereby creating a positively charged quaternary nitrogen. The present invention encompasses utilizing organostannanes which are so substituted and charged. It is understood that when such a charged organostannane is utilized the product resulting from the instant process will also be similarly charged. It is also understood that, when such a charged species is utilized or produced, a corresponding negatively charged counterion must also be present with the compound or the compound itself must contain a negatively charged substituent, thereby making the compound neutral in toto.

It is understood that the substituent (or substituents) on the $R^3$ group of the organostannane employed may also contain a positively charged quaternary nitrogen. For example, 4-(trimethylammoniumethyl)-1-trimethylstannylbenzene chloride and the like may be utilized in the instant process. In such a case the product produced by the instant process will also be similarly charged. It is understood that when such stannanes are utilized and such products produced, a negatively charged counterion or a negatively charged substituent must be present as noted hereinabove.

It is preferred that R is hydrogen.

It is preferred that $R^2$ is hydrogen and $R^1$ is (*R*)-$CH_3CH(OR^4)$- or (*R*)-$CH_3CH(F)$-,and (***R***)-$CH_3CH(OR^4)$- is most preferred.

It is preferred that $R^3$ is substituted dibenzofuranyl, phenanthrenyl, dibenzothienyl, phenyl, biphenyl, naphthyl, fluoren-9-onyl, 9-oxodibenzothienyl or 9,9-dioxodibenzothienyl.

It is preferred that leaving group X is trifluoromethanesulfonyloxy or chloro. The most preferred group X is trifluoromethanesulfonyloxy.

It is preferred that $R^s$ is methyl or butyl. The most preferred $R^s$ is methyl.

It is preferred that 1.0 to 1.2 molar equivalents of the aryl stannane is employed in the process. It is more preferred that 1.0 to 1.1 molar equivalents of the aryl stannane is employed in the process.

It is preferred that $R^p$ is a phenyl group appropriately substituted 1 to 3 times with an electron-donating group such as methyl, methoxy, ethoxy and the like. The most preferred $R^p$ is 2,4,6-trimethoxyphenyl.

It is preferred that 0 (zero) to 4 molar equivalents, with respect to the palladium compound, of the phosphine is employed in the process.

It is preferred that the palladium compound is $Pd_2(DBA)_3$·solvent or $(CH_3CN)_2PdCl_2$. The most preferred palladium compound is $Pd_2(DBA)_3$·$CHCl_3$.

It is preferred that 2 to 6 mole percent of the palladium compound is employed in the process. It is more preferred that 2 to 4 mole percent of the palladium compound is employed in the process.

It is preferred that the halide source is lithium chloride, zinc chloride or a substituted ammonium halide. The most preferred halide source is zinc chloride.

It is preferred that 1.0 to 2.5 molar equivalents of the halide source is employed in the process. It is more preferred that 1.0 to 1.5 molar equivalents of the halide source is employed in the process.

It is preferred that the aprotic polar coordinating solvent is tetrahydrofuran, dimethylformamide or 1-methyl-2-pyrrolidinone or a mixture of any of the three. The most preferred aprotic polar coordinating solvent is 1:1 1-methyl-2-pyrrolidinone:tetrahydrofuran.

The carbapenem compound I produced by the process of the present invention is useful per se as a synthetic intermediate which leads directly or indirectly to carbapenem antibiotics. It is understood that the protecting group Y and/or $R^5$ may be removed by methodology well known in the art to directly provide the corresponding carbapenem antibiotic.

It is also understood that compounds of Formula I may subsequently be further modified prior to the removal of the protecting group Y. Such chemical modifications may lead to carbapenem antibiotics, such as those disclosed in U.S. Pat. Nos. 4,680,292 and 4,978,659, EPO Publ. Nos. 0277743, 0414489, 0414492 and 0414493, and U.S. Pat. Appls. SN 543939, 544281 and 546279, and U.S. Pat. Appls. Merck Docket No. 17510, 17511, 17805, 18095 and 18136 all assigned to Merck & Co., Inc., which may have greater antibacterial potency than compounds obtained directly from the deprotection of carbapenems of Formula I. Possible modification processes are disclosed in the cited U.S. Patent, EPO and U.S. Pat. Appls., but are not meant to be limiting.

The carbapenem antibiotics so produced are valuable antibacterial agents active against various Gram-positive and Gram-negative bacteria and accordingly find utility in human and veterinary medicine. Representative pathogens which are sensitive to the antibacterial agents of the present invention include various species or strains of the following: Staphylococcus, Enterococcus, Escherichia coli, Klebsiella, Enterobacter, Bacillus, Salmonella, Pseudomonas, Serratia, Proteus, and Bacterium. Such antibiotics are not limited to utility as medicaments; they may be used in all manner of industry, for example: additives to animal feed, preservation of food, disinfectants, and in other industrial systems where control of bacterial growth is desired. The U.S. Patents and Patent Aplications referred to hereinabove further describe the utility of such compounds.

## DETAILED DESCRIPTION OF THE INVENTION

The following synthetic scheme (Scheme 1) illustrates the sequence in which the process of the present invention is employed. The various substituents R, $R^1$, $R^2$, $R^3$ and Y are as defined above. The substituent M is defined hereinbelow.

SCHEME 1

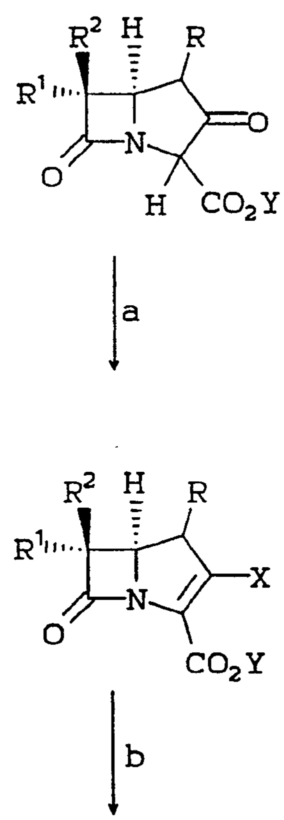


V

---

a. activation

b. coupling

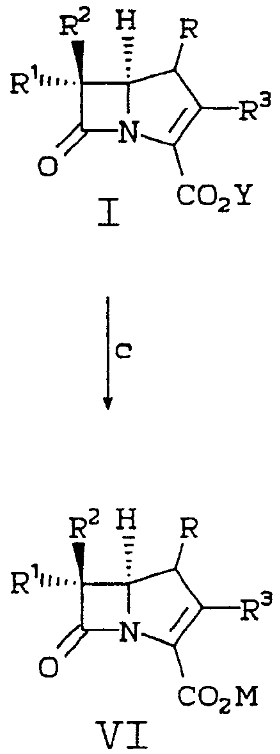


I

VI

c. deprotection

The steps for preparing the 2-oxocarbapenam intermediate V are well known in the art and are explained in ample detail by D.G. Melillo et al., Tetrahedron Letters, **21**, 2783(1980), T. Salzmann et al., J. Am. Chem. Soc., **102**, 6161(1980), J. G. deVries et al., Heterocycles, **23** (8), 1915 (1985); and L. M. Fuentes, I. Shinkai, and T. N. Salzmann, J. Am. Chem. Soc., **108**, 4675 (1986). The syntheses are also disclosed in Sanraku Ocean Japanese patent publication No. 6-0163-882-A, Sandoz Belgium patent publication No. 900-718-A, and U.S. Pat. No. 4.269,772, U.S. Pat. No. 4,350,631, U.S. Pat. No. 4,383,946 and U.S. Pat. No. 4,414,155 all assigned to Merck & Co., Inc. The above papers and disclosures are incorporated herein by reference.

In words relative to the equations, the 2-oxocarbapenam V, which has a protecting group Y, such as triorganosilyl, substituted and unsubstituted allyl and substituted and unsubstituted benzyl groups, and may also have related protecting groups incorporated in the substituents $R^1$ and $R^2$, is converted to a carbapenem having a leaving group at the 2-position. A choice of several leaving groups known in the art may be employed. Thus the oxocarbapenam may be reacted with a phosphorylating agent, such as diphenylchlorophosphate, in the presense of an organic nitrogen base, such as diisopropylethylamine and the like, to provide an enol phosphate (e.g.,X=OPO(OPh)$_2$). This procedure is described by Melillo et al., supra and Salzmann et al., supra. Alternatively the oxocarbapenam may be reacted with a sulfonylating agent, such as methanesulfonyl chloride, trifluoromethanesulfonyl chloride, trifluoromethanesulfonic anhydride and the like, in the presence of an organic nitrogen base, such as diisopropylamine, triethylamine, and the like, to provide an enol sulfonate (e.g., X=OTf). As a final alternative the enol sulfonate so produced may be treated with a metal halide, such as lithium chloride and the like (or, alternatively, with a metal halide in the presence of a palladium compound and optionally a phosphine ligand), to provide a vinyl halide carbapenem (e.g., X=Cl).

The 2-activated-carbapenem is then subsequently reacted with an organotin reagent, such as phenyltrimethylstannane, 4-formylphenyltrimethylstannane and the like, in the presence of a catalyst, such as $Pd(DBA)_2$, $Pd_2(DBA)_3.CHCl_3$, $(CH_3CN)_2PdCl_2$ and the like. Such a coupling reaction may benefit from the presence of a metal halide and may also benefit from the presence of a phosphine ligand, such as a triarylphosphine and the like, and provides a protected carbapenem of the formula I.

The carbapenem I is then deprotected by procedures known in the art, dependent on which protecting groups are present in I, to provide the carbapenem antibiotic VI. Thus, if Y is an substituted or unsubstituted allyl group and/or an allylcarbonyl type protecting group is present in $R^1$ or $R^2$, a palladium catalyzed de-esterification, such as described by McCombie et al., J. Org. Chem., **47**, 2505(1983), may be employed. Alternatively, if a substituted or unsubstituted benzyl protecting group is present in carbapenem I, such a group may be removed by well known hydrogenation techniques, such as described by Melillo et al., ***supra***. And further, if a triorganosilyl group is present in carbapenem I, such a group may be removed by well known protic or aprotic desilylation techniques, such as described by R. Guthikonda et al., ***supra***. It is understood that if combinations of protecting groups are present the sequence by which the protecting groups are removed would be dictated by factors such as solubility and stability of the "partially protected" carbapenem and could be easily determined by one skilled in the art.

As used herein M is hydrogen or an alkali metal. The term "alkali metal" includes potassium, sodium and lithium.

Scheme 2 illustrates a sequence in which the process of the present invention is employed to provide the intermediate Ia wherein $R^1$ is (***R***)-$CH_3CH(OR^4)$-, $R^2$ is hydrogen, X is trifluoromethanesulfonate and $R^4$ is hydrogen or triorganosilyl, these being the preferred embodiments. The various other substituents R, $R^3$, Y and M are as defined hereinabove. It is understood that the optional organosilyl protecting group $R^5$ ($R^4$=$R^5$=triorganosilyl may be introduced prior to the reaction of 2-oxocarbapenam Va with the trifluoromethanesulfonylating agent, but the sequence illustrated is found to be more convenient since it is performed in a single reaction vessel.

SCHEME 2

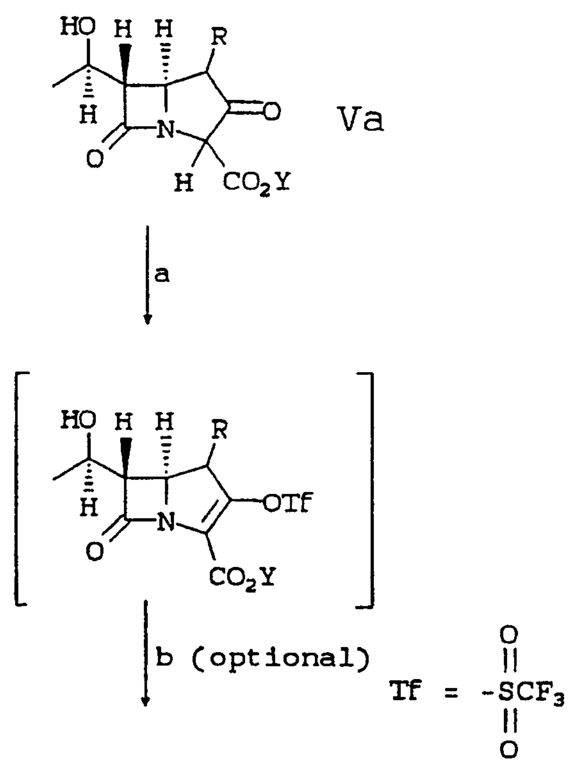


Va

a. trifluoromethanesulfonating agent

base/aprotic solvent

b. silylating agent/base

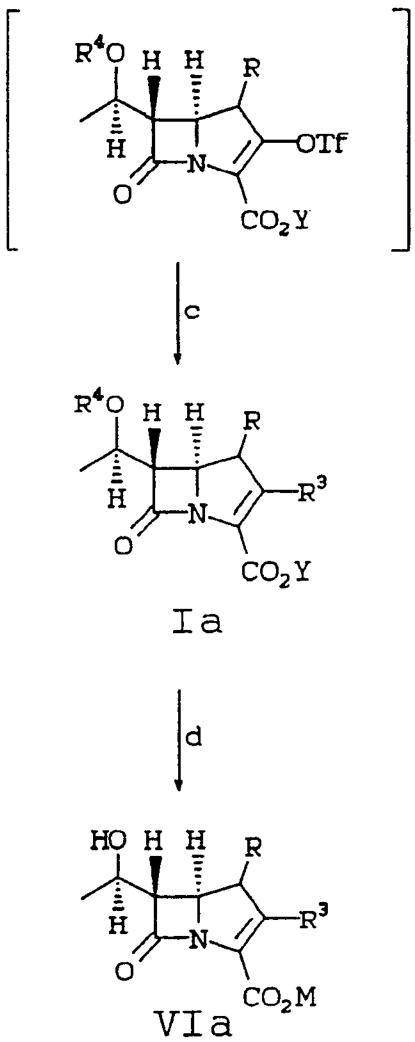


Ia

VIa

c. palladium compound/metal halide

aprotic polar coord. solvent

$R^8{}_3Sn{-}R^3$

III

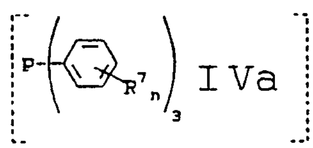

IVa

d. deprotection

In words relative to the equations, the 2-oxocarbapenam Va, which has a protecting group Y, such as triorganosilyl, substituted and unsubstituted allyl and substituted and unsubstituted benzyl groups, is reacted with a suitable trifluoromethanesulfonyl source, such as trifluoromethanesulfonic anhydride, trifluoromethanesulfonyl chloride and the like, in the presence of a organic nitrogen base, such as triethylamine, diisopropylamine and the like, in a polar aprotic solvent, such as tetrahydrofuran. If it is desirable to protect the hydroxyethyl group in the 6-position of the carbapenem, an organic nitrogen base, such as triethylamine and the like, is then added to the reaction solution followed immediately by a silylating agent, such as trimethylsilyl trifluoromethanesulfonate, triethylsilyl trifluoromethanesulfonate and the like. Whether such protection of the hydroxyl group is or is not employed, the reaction is allowed to stir for a brief time and then an aprotic polar coordinating solvent, such as DMI, 1-methyl-2-Pyrrolidinone and the like, is added. This is followed by the addition of a palladium compound, such as tris(dibenzylideneacetone)dipalladium- chloroform, palladium acetate and the like, a suitably substituted stannane, such as 1-(trimethylstannyl)-4-(hydroxymethyl)benzene and the like and, optionally, a

suitably substituted phenylphosphine ($R^7$=lower alkyl or lower alkoxy; n=0 to 3), such as tris(4-methoxyphenyl)phosphine, tris(2,4,6-trimethoxyphenyl)-phosphine and the like. A metal halide, such as lithium chloride, zinc chloride and the like, is added and the reaction solution is quickly warmed to a suitable temperature, such as 0° to 50°C, and allowed to stir for a suitable ammount of time. The product carbapenem Ia is obtained by conventional isolation/purification methodology known in the art.

As previously described hereinabove carbapenem Ia is deprotected with the appropriate technique to provide the carbapenem antibiotic VI.

Scheme 3 illustrates a sequence in which the process of the present invention is employed to provide the intermediate Ia wherein R is hydrogen, $R^1$ is (*R*)-$CH_3CH(OR^5)$-, $R^2$ is hydrogen, X is trifluoromethanesulfonate, Y is p-nitrobenzyl and $R^5$ is trimethylsilyl, these being the most preferred embodiments. The other substituent $R^3$ is as defined hereinabove. The specific preferred reagents are also illustrated in Scheme 3.

SCHEME 3

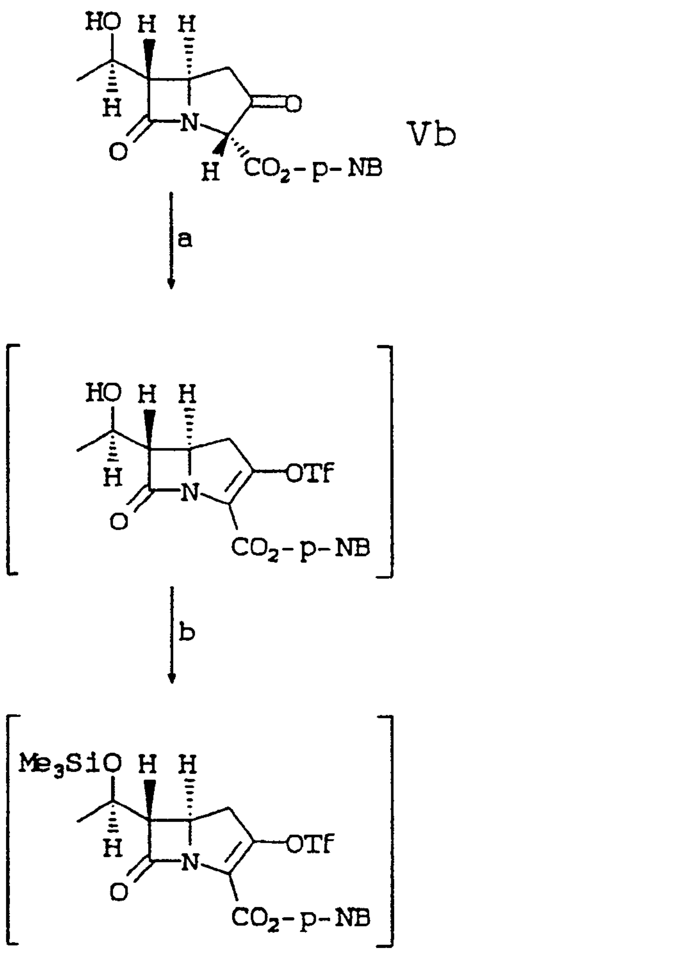


Vb

a. $iPr_2NH/Tf_2O/THF$

b. $Et_3N/TMSOTf$

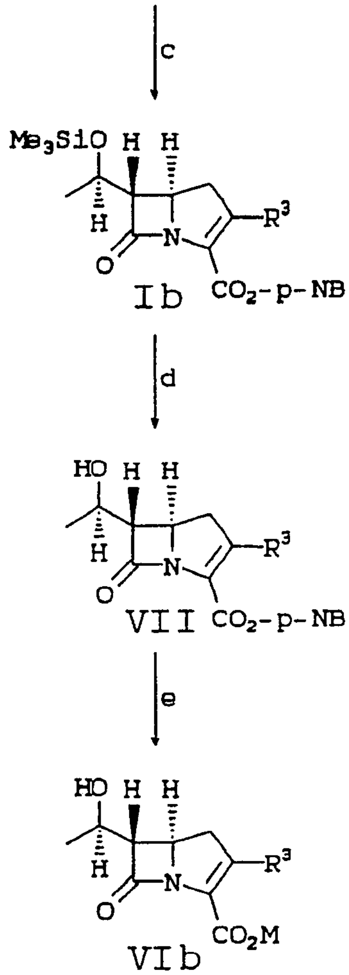


Ib

VII

VIb

c. $Pd_2(DBA)_3 \cdot CHCl_3/ZnCl_2$

1-methyl-2-pyrrolidinone

$Me_3Sn—R^3$

III

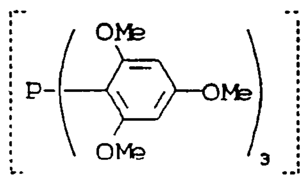

d. AcOH

e. $H_2/KHCO_3/10\%$ Pd on C

In words relative to the equations, the 2-oxocarbapenam Vb is reacted with diisopropylamine in tetrahydrofuran at a temperature of -78°C, followed in 10 min. by trifluoromethanesulfonic anhydride. The reaction is allowed to stir for 15 min., and then triethylamine is added to the reaction solution followed immediately by trimethylsilyl trifluoromethanesulfonate. The reaction is allowed to stir for 20 min., and then 1-methyl-2-pyrrolidinone is added followed by tris(dibenzylideneacetone)dipalladium-chloroform and a suitably substituted organostannane, such as 1-(trimethylstannyl)-4-(hydroxymethyl)-benzene and the like, and, optionally, tris(2,4,6-trimethoxyphenyl)-phosphine. A solution of zinc chloride in ether is added and the reaction solution is quickly warmed to a suitable temperature, such as 0° to 50°C, and allowed to stir for a suitable ammount of time. The product carbapenem Ib is obtained by conventional isolation/purification methodology known in the art.

Carbapenem Ib is then reacted with acetic acid to provide the 6-hydroxyethylcarbapenem VII. Without isolation, carbapenem VII is hydrogenated over 10% Pd on carbon in the presence of potassium bicarbonate to provide the carbapenem antibiotic VIb.

It is understood that the synthetic process described hereinabove is compatible with other organostannanes not specifically described. Thus organostannanes having a (heteroarylium)methylphenyl group or a heteroaryliumalkyl group as $R^3$ could be utilized under the same reaction conditions to provide the direct precursors to the carbapenem antibiotics disclosed in U.S. Pat. Nos. 4,680,292, 4,729,993 and 4,978,659, and EPO Publ. Nos. 0277743, 0414489, 0414492 and 0414493, all assigned to Merck & Co. Inc.

The invention is further defined by reference to the following examples, which are intended to be illustrative and not limiting. All temperatures are in degrees Celsius.

EXAMPLE 1

p-Nitrobenzyl-(5*R*,6*S*)-2-(1-carbamoyl-3-dibenzofuranyl) -6-[1*R*-(trimethylsilyloxy)ethyl]carbapen-2-em-3-carboxylate

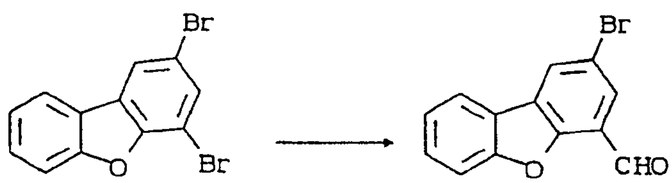

1 2

Step A: 1-Formyl-3-bromodibenzofuran

To a stirred solution of the dibromobenzofuran **1** (10g, 30.9 mmol) in anhydrous THF (250 mL) at -78°C under nitrogen was added a 2.5M butyllithium in hexanes solution (13.6 mL, 33.9 mmol). The resulting red solution was warmed to -50°C and held there for 10 min. before anhydrous DMF (2.6 mL, 33.9 mmol) was added dropwise. The resulting rust colored solution was stirred an additional 20 min. at -50° to -40°C before being quenched with saturated ammonium chloride solution (25 mL). The THF was removed under vacuum and the residue was dissolved in ethyl acetate (EtOAc) and washed sequentially with water, saturated aqueous ammonium chloride solution, water and brine. The organic solution was then dried with magnesium sulfate and decolorized with Norite. The mixture was then filtered and concentrated under vacuum. The residue was triturated with ether/hexane to provide 4.0 g of pale yellow flakes of dibenzofuran **2**. The mother liquor was then chromatographed (silica gel, 30% EtOAc in hexanes) to provide an additional 2.1 g of dibenzofuran **2** (total yield: 73%).

$^1$H-NMR (300 MHz, $CDCl_3$): δ 7.42 (t, J=7.5 Hz, 1H), 7.55 (t, J=7.3 Hz, 1H), 7.65 (d, J=7.9 Hz, 1H), 7.92 (d, J=7.7 Hz, 1H), 8.02 (d, J=1.6 Hz, 1H), 8.25 (d, J=1.9 Hz, 1H), 10.51 ppm (s, 1H).

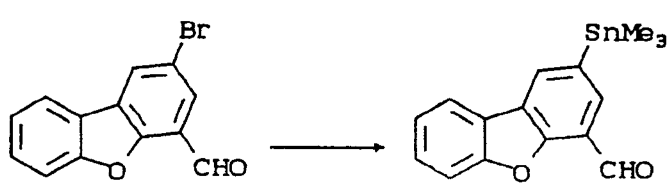

Step B: 1-Formyl-3-(trimethylstannyl)dibenzofuran

To a stirred solution of the dibenzofuran **2** from Step A (5 g, 18.2 mmol) in toluene (91 mL) was added hexamethylditin (3.9 mL, 20 mmol), tetrakis(triphenylphosphine)palladium (0) (1.05 g, 5 mol %) and triphenylphosphine (0.276 g, 5 mol %). Nitrogen was bubbled through the solution for 5 min., and the reaction solution was heated at reflux for 15 min. under a nitrogen atmosphere. The reaction mixture was then poured into ether and the organic solution was washed with water (3 times) and then brine (2 times). The solution was dried with magnesium sulfate, filtered and concentrated under vacuum. The residue was purified by flash chromatography (silica gel, 5% EtOAc in $CH_2Cl_2$) and crystallized to provide 4.3 g (66% yield) of stannane **3** as a white solid.

$^1$H-NMR (300 MHz, $CDCl_3$): δ 0.40 (s, 9H), 7.40 (t, J=6.3 Hz, 1H), 7.52 (t, J=6.3 Hz, 1H), 7.68 (d, J=6.1 Hz,

1H), 8.00 (m, 2H), 8.19 (s, 1H), 10.62 ppm (s, 1H).

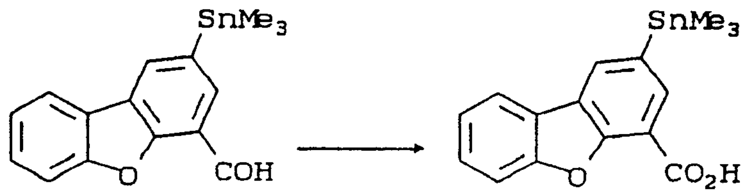

Step C: 1-Carboxy-3-(trimethylstannyl)dibenzofuran

A solution of tetra-n-butylammonium permanganate (5.1 g, 14.0 mmol) in anhydrous pyridine (35 mL) was transferred via cannula needle into a solution of the stannane **3** from Step B (5.0 g, 14.0 mmol) in anhydrous pyridine (35 mL) at 0°C under a nitrogen atmosphere. The reaction was stirred for 30 min., then saturated aqueous sodium sulfate (50 mL) was added to quench the reaction. The mixture was then poured into ether and the layers separated. The organic layer was washed with 2N aqueous HCl (6 times with 100 mL), water (2 times) and then brine (2 times). The solution was dried with magnesium sulfate, then filtered and concentrated under vacuum to provide 4.8 g (92% yield) of the stannane **4** as a white solid.
$^1$H-NMR (300 MHz, $CDCl_3$): δ 0.40 (s, 9H), 7.39 (t, J=8.4 Hz, 1H), 7.52 (t, J=8.4 Hz, 1H), 7.71 (d, J=8.4 Hz, 1H), 8.00 (d, J=7.8 Hz, 1H), 8.27 (s, 1H), 8.29 ppm (s, 1H).

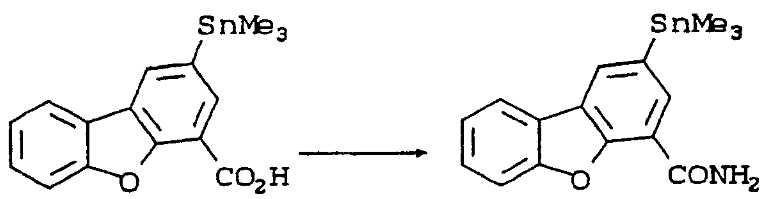

4 5

Step D: 1-Carbamoyl-3-(trimethylstannyl)dibenzofuran

To a stirred solution of the stannane **4** from Step C (1.1 g, 2.96 mmol) in anhydrous acetonitrile (5 mL) and THF (15 mL) under a nitrogen atmosphere was added 1-(3-dimethylaminopropyl)-3-ethylcarbodiimide hydrochloride (1.13 g, 5.9 mmol) and 1-hydroxybenztriazole hydrate (1.2 g, 8.9mmol). The solution was stirred 30 min., then 11 mL of a 2.6M ethanolic ammonia solution was added. The resulting milky white solution was stirred an additional 30 min. before being quenched with saturated aqueous ammonium chloride. The solvents were removed under vacuum and the residue taken up in ether (75 mL) and EtOAc (75 mL). The solution was washed with water (3 times) and brine (2 times), then dried with magnesium sulfate and filtered. The solution was concentrated under vacuum and the residue was purified by flash chromatography (silica gel, 35% EtOAc in hexanes) to provide 979 mg (88% yield) of stannane **5** as a white solid.
$^1$H-NMR (300 MHz, $CDCl_3$): δ 0.38 (s, 9H), 6.10 (broad s, 1H), 7.41 (t, J=7.2 Hz, 1H), 7.49 (t, J=7.2 Hz, 1H), 7.54-7.66 (m, 2H), 7.99 (d, J=7.8 Hz, 1H), 8.22 (s, 1H), 8.35 ppm (s, 1H).

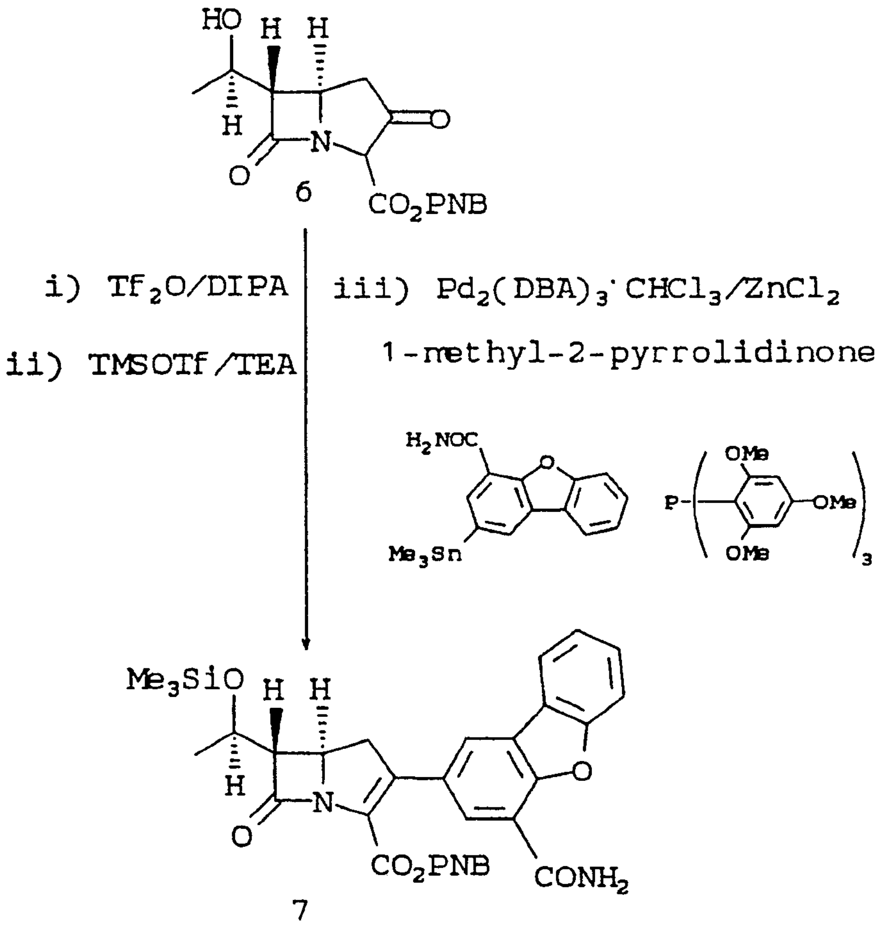


6

7

Step E: p-Nitrobenzyl-(5*R*,6*S*)-2-(1-carbamoyl-3-dibenzofuranyl) -6-[1*R*-(trimethylsilyloxy) ethyl]carbapen-2-em-3-carboxylate

A dry 15 mL receiving flask was charged with the bicyclic β-keto carbapenam ester **6** (143 mg; 0.41 mmol) and a magnetic stir bar and the system was purged with nitrogen. Two mL of anhydrous tetrahydrofuran (THF) was added and upon dissolution, the reaction vessel was cooled to -78°C under $N_2$. Diisopropylamine (0.063 mL, 0.45 mmol) was then added and the stirring was continued for 10 minutes.

Trifluoromethanesulfonic anhydride (0.075 mL, 0.45 mmol) was added, followed by stirring for an additional 15 mins. Triethylamine (0.062 mL, 0.45 mmol) was then added, followed by trimethylsilyltrifluoromethanesulfonate (0.087 mL, 0.45 mmol).

While the above reaction was stirred for 20 mins., the organostannane **5** from Step D (168 mg, 0.45 mmol), tris(dibenzylideneacetone)dipalladium-chloroform (8.5 mg, 0.0082 mmol) and tris(2,4,6-trimethoxyphenyl)phosphine (17.4 mg, 0.033 mmol) were weighed into a single vial and the vial was purged with nitrogen. When the above reaction time had elapsed, N-methylpyrrolidinone (2mL) was added to the initial reaction mixture followed by the previously weighed solids. A 0.87M zinc chloride in ether solution (0.52 mL, 0.45 mmol) was then added.

The low temperature bath was then removed and the reaction vessel was placed in a luke warm water bath to allow it to quickly reach ambient temperature. After reaching ambient temperature, the mixture was stirred for 20 minutes.

The reaction was then quenched by pouring the contents of the flask into a 125 mL separatory funnel containing diethyl ether, ethyl acetate and water. The organic phase was separated and washed with water and brine. The organic phase was dried over magnesium sulfate. The mixture was then filtered and the solvent removed under vacuum. Flash column chromatography of the residue (silica gel, 60-65% ethyl acetate/hexanes) provided 164 mg (67%) of carbapenem **7** as a slightly yellowish foam. $^1$H-NMR (300 MHz, $CDCl_3$): δ 0.15 (s, 9H), 1.30 (d, J=6.2 Hz, 3H), 3.28 (dd, J=6.4, 2.7 Hz,1H), 3.31-3.45 (m, 2H), 4.21-4.35 (complex m, 2H), 5.21 ($AB_q$, $J_{AB}$=13.5 Hz, $\Delta\nu_{AB}$=50.1 Hz, 2H), 6.17 (broad singlet, 2H), 7.35-7.41 (m, 3H), 7.48-7.54 (m, 1H), 7.60 (d, J=8.3 Hz, 1H), 7.83 (d, J=7.2 Hz, 1H), 7.97 (d, J=8.8 Hz, 2H), 8.09 (d, J=2.0 Hz, 1H), 8.18 ppm (d, J=1.9 Hz, 1H); IR ($CHCl_3$) 3510, 3400, 1770, 1720, 1675, 1590, 1520 $cm^{-1}$; U.V. ($CH_3CN$): $\lambda_{max}$ 290 nm (ε

11,000); $\lambda_{max}$ 250nm (ε 13,300)

The following Examples were performed on the same scale and under the conditions as described for Step E of Example 1, except as otherwise noted. Each of the following Examples will list the organostannane which was substituted for stannane **5** in Example 1, the yield of the reaction and the pertinent physical and spectroscopic data. If the synthesis of the organostannane employed is not known in the art that synthesis is included in the Example or the synthesis described in Example 1, Step B, may be employed but substituting appropriate aryl halides known in the art for the dibenzofuran 2 of Example 1, Step B.

EXAMPLE 2

p-Nitrobenzyl-(5***R***,6***S***)-2-phenyl-6-[1***R***-(trimethylsilyl-oxy)ethyl]carbapen-2-em-3-carboxylate

Trimethylstannylbenzene 73%.
$^1$H-NMR (300 MHz, $CDCl_3$): δ 0.13 (s, 9H), 1.28 (d, J=6.2 Hz, 3H), 3.14-3.28 (complex m, 3H), 4.21-4.29 (complex m, 2H), 5.26 ($AB_q$, $J_{AB}$=14.0 Hz, $\Delta\nu_{AB}$=50.2 Hz, 2H), 7.32 (s, 5H), 7.40 (d, J=8.8 Hz, 2H), 8.13 ppm (d, J=8.7 Hz, 2H);
IR ($CHCl_3$) 1770, 1720, 1601, 1520 $cm^{-1}$; U.V. ($CH_3CN$): $\lambda_{max}$ 267 nm (ε 9,800).

EXAMPLE 3

P-Nitrobenzyl-(5***R***,6***S***)-2-(4-methoxyphenyl) -6-[1***R***-(trimethylsilyl-oxy)ethyl]carbapen-2-em-3-carboxylate

4-Trimethylstannylanisole 64%.
$^1$H-NMR (300 MHz, $CDCl_3$): δ 0.13 (s, 9H), 1.28 (d, J=6.2 Hz, 3H), 3.13-3.24 (complex m, 3H), 3.78 (s, 3H), 4.18-4.25 (complex m, 2H), 5.27 ($AB_q$, $J_{AB}$=14.0 Hz, $\Delta\nu_{AB}$=56.7 Hz, 2H), 6.83 (d, J=8.9 Hz, 2H), 7.35 (d, J=9.0 Hz, 2H), 7.50 (d, J=8.7 Hz, 2H), 8.14 ppm (d, J=8.8 Hz, 2H);
IR ($CHCl_3$) 1770, 1718, 1600, 1520, 1510 $cm^{-1}$; U.V. ($CH_3CN$): $\lambda_{max}$ 318 nm (ε 11,600), $\lambda_{max}$ 267 nm (ε 13,700); m.p.=121°C.

EXAMPLE 4

P-Nitrobenzyl-(5***R***,6***S***) -2-(2-methoxyphenyl)-6-[1***R***-(trimethylsilyl-oxy)ethyl]carbapen-2-em-3-carboxylate

2-Trimethylstannylanisole, 82%.
$^1$H-NMR (300 Hz, $CDCl_3$): δ 0.13 (s, 9H), 1.29 (d, J=6.2 Hz, 3H), 3.10 (dd, J=18.4,10.0 Hz, 1H), 3.22-3.34 (complex m, 2H), 3.73 (s, 3H), 4.21-4.29 (complex m, 2H), 5.20 ($AB_q$, $J_{AB}$=14.0 Hz, $\Delta\nu_{AB}$=43.0 Hz, 2H), 6.84-6.93 (m, 2H), 7.12 (dd, J=7.6,1.7 Hz, 1H), 7.24-7.34 (m, 3H), 8.07 ppm (d, J=8.8 Hz, 2H);
IR ($CHCl_3$) 1770, 1720, 1603, 1520 $cm^{-1}$; U.V. ($CH_3CN$): $\lambda_{max}$ 270 nm (ε 12,500).

EXAMPLE 5

p-Nitrobenzyl-(5***R***,6***S***) -2-(4-acetylphenyl)-6-[1***R***-(trymethylsilyl-oxy)ethyl]carbapen-2-em-3-carboxylate

4-(Trimethylstannyl)acetophenone, 77%.
$^1$H-NMR (300 MHz, $CDCl_3$): δ 0.13 (s, 9H), 1.28 (d, J=6.2 Hz, 3H), 2.58 (s, 3H), 3.19 (dd, J=17.4,9.6 Hz, 1H), 3.25-3.38 (complex m, 2H), 4.19-4.34 (complex m, 2H), 5.27 ($AB_q$, $J_{AB}$=13.8 Hz, $\Delta\nu_{AB}$=50.2 Hz, 2H), 7.42 (d, J=8.4 Hz, 2H), 7.51 (d, J=8.6 Hz, 2H), 7.90 (d, J=8.4 Hz, 2H), 8.15 ppm (d, J=8.7 Hz, 2H);
IR ($CHCl_3$) 1775, 1724, 1680, 1600, 1520 $cm^{-1}$; U.V. ($CH_3CN$): $\lambda_{max}$ 253 nm (320 shoulder), (ε 13,500).

EXAMPLE 6

p-Nitrobenzyl-(5***R***,6***S***)-2-(4-formylphenyl) -6-[1***R***-(trimethylsilyl-oxy)ethyl]carbapen-2-em-3-carboxylate

1-(Trimethylstannyl)benzaldehyde, 73%.
$^1$H-NMR (300 MHz, $CDCl_3$): δ 0.13 (s, 9H), 1.28 (d, J=6.2 Hz, 3H), 3.18 (dd, J=18.2,10.1 Hz, 1H), 3.27 (dd, J=6.0,2.9Hz, 1H), 3.34 (dd, J=18.3,8.9Hz, 1H), 4.20-4.34 (complex m, 2H), 5.28 ($AB_q$, $J_{AB}$=13.8 Hz, $\Delta\nu_{AB}$=49.7 Hz, 2H), 7.50 (apparent t, J=8.4 Hz, 4H), 7.83 (d, J=8.3 Hz, 2H), 8.15 ppm (d, J=8.7 Hz, 2H), 9.99 (s, 1H);
IR ($CHCl_3$) 1775, 1720, 1700, 1600, 1520 $cm^{-1}$; U.V. ($CH_3CN$):$\lambda_{max}$ 325 nm (ε 13,500); $\lambda_{max}$ 254 nm (ε 20,300).

EXAMPLE 7

p-Nitrobenzyl-(5*R*,6*S*)-2-(4-cyanophenyl)-6-[1*R*-(trimethylsilyloxy)ethyl]carbapen-2-em-3-carboxylate

4-(Trimethylstannyl)benzonitrile, 70%.
$^1$H-NMR (300 MHz, $CDCl_3$): δ 0.13 (s, 9H), 1.28 (d, J=6.2 Hz, 3H), 3.18 (dd, J=18.2,10.1 Hz, 1H), 3.25-3.37 (complex m, 2H), 4.19-4.32 (complex m, 2H), 5.28 ($AB_q$, $J_{AB}$=13.8 Hz, $\Delta\nu_{AB}$=50.9 Hz, 2H), 7.45 (d, J=8.5 Hz, 2H), 7.54 (d, J=8.8 Hz, 2H), 7.62 (d, J=8.6 Hz, 2H), 8.18 ppm (d, J=8.7 Hz, 2H);
IR ($CHCl_3$) 2212, 1778, 1723, 1601, 1520 $cm^{-1}$; U.V. ($CH_3CN$):$\lambda_{max}$ 315 nm (ε 9,000), $\lambda_{max}$ 265 nm (ε 11,800), $\lambda_{max}$ 235 nm (ε 12,800).

EXAMPLE 8

P-Nitrobenzyl-(5*R*,6*S*)-2-(3-biphenyl)-6-[1*R*-(trimethylsilyloxy)ethyl]carbapen-2-em-3-carboxylate

3-Trimethylstannylbiphenyl, 38%.
$^1$H-NMR (300 MHz, $CDCl_3$): δ 0.14 (s, 9H), 1.29 (d, J=6.2 Hz, 3H), 3.19-3.40 (complex m, 3H), 4.20-4.31 (complex m, 2H), 5.21 ($AB_q$, $J_{AB}$=14.0 Hz, $\Delta\nu_{AB}$=49.8 Hz, 2H), 7.28-7.58 (complex m, 11H), 8.05 ppm (d, J=8.7 Hz, 2H);
IR ($CHCl_3$) 1770, 1720, 1600, 1520 $cm^{-1}$;
U.V. ($CH_3CN$):$\lambda_{max}$ 257 nm (ε 13,100).

EXAMPLE 9

P-Nitrobenzyl-(5*R*, 6*S*) -2-(fluoren-9-on-2-yl)-6-[1*R*-(trimethylsilyloxy)ethyl]carbapen-2-em-3-carboxylate

2-(Trimethylstannyl)fluoren-9-one, 73%
$^1$H NMR (300 MHz, $CDCl_3$): δ 0.13 (s, 9H), 1.28 (d, J=6.2 Hz, 3H), 3.15-3.38 (complex m, 3H), 4.21-4.31 (complex m, 2H), 5.26 (ABq, $J_{AB}$=13.6 Hz, $\Delta\nu_{AB}$ = 53.7 Hz, 2H), 7.26-7.34 (m, 1H), 7.41-7.63 (complex m, 8H), 8.09 ppm (d, J=8.7 Hz, 2H);
IR ($CHCl_3$) 1770(s), 1720(s), 1610(m), 1600(m), 1520(m) $cm^{-1}$;
U.V. ($CH_3CN$): $\lambda_{max}$ = 257; ε = 22,600.

EXAMPLE 10

p-Nitrobenzyl-(5*R*,6*S*) -2-(7-hydroxymethyl-3-dibenzofuranyl)-6-[1*R*-(trimethylsilyloxy)ethyl]carbapen-2-em-3-carboxylate

Method 1:

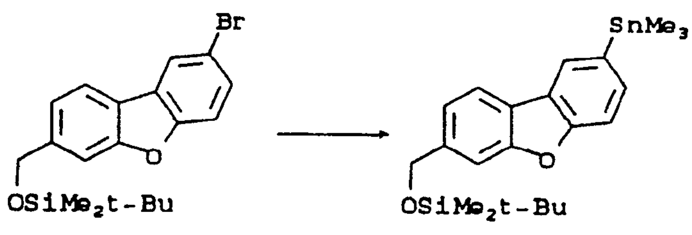

8 9

Step A: 3-(Trimethylstannyl)-7-(t-butyldimethylsilyl-oxymethyl)dibenzofuran

To a solution of the dibenzofuran 8 (995 mg, 2.5 mmol) in anhydrous THF (25 mL) at -78°C under a nitrogen atmosphere was added a 1.5M t-butyllithium in pentane solution (3.0 mL, 5.25 mmol). The resulting yellow solution was stirred for 100 min., then trimethyltin chloride (548 mg, 2.75 mmol) was added as a solid. The mixture was allowed to warm to ambient temperature and then stirred for 3 hours. The reaction mixture was then poured into ether and the organic solution was washed with water (3 times) and then with brine. The organic solution

was then dried with magnesium sulfate, filtered and concentrated under vacuum. Flash chromatography of the residue (silica gel, 10% methylene chloride in hexanes) provided 815 mg of the stannane **9** (68% yield) as a crystalline solid.
$^1$H-NMR (300 MHz, $CDCl_3$): δ 0.22 (s, 6H), 0.35 (s, 9H), 0.95 (s, 9H), 4.88 (s, 2H), 7.24-7.28 (m, 1H), 7.52-7.59 (m, 2H), 7.89 (d, J=7.2 Hz, 1H), 8.02 ppm (s, 1H).

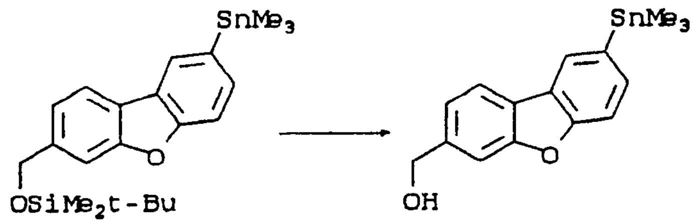

9 10

Step B: 3-(Trimethylstannyl)-7-(hydroxymethyl)-dibenzofuran

To a solution of the dibenzofuran **9** of Step A (339 mg,0.71 mmol) in anhydrous THF (7 mL) at 0°C under a nitrogen atmosphere was added dropwise a 1M solution of tetrabutylammonium fluoride in THF (0.92 mL, 0.92 mmol). The reaction solution was stirred for 30 min., then saturated ammonium chloride was added. The mixture was then extracted with EtOAc and the organic solution was washed with brine. The organic solution was then dried with magnesium sulfate and then filtered and concentrated under vacuum. Flash chromatograhy of the residue (silica gel, 25% EtOAc in hexanes) provided 182 mg of **10** (70% yield) as a white solid.
$^1$H-NMR (300 MHz, $CDCl_3$): δ 0.35 (s, 9H), 1.75 (apparent t, J=5.0 Hz, 1H), 4.85 (d, J=5.9 Hz, 2H), 7.34 (d, J=7.8 Hz, 1H), 7.52-7.60 (m, 3H), 7.84 (d, J=7.8 Hz, 1H), 8.05 ppm (s, 1H).

Step C: p-Nitrobenzyl-(5*R*,6*S*) -2-(7-hydroxymethyl-3-dibenzofuranyl)-6-[1*R*-(trimethylsilyloxy)-ethyl]carbapen-2-em-3-carboxylate

Using the procedure described in Step E of Example 1 but substituting the stannane **10** for the stannane **5** in Example 1 provided the title compound in 70% yield.
$^1$H-NMR (300 MHz, $CDCl_3$): δ 0.15 (s, 9H), 1.30 (d, J=6.3 Hz, 3H), 1.97 (dd, $J_1$=$J_2$=3.0 Hz,1H), 3.27 (dd, J=6.4, 2.9 Hz,1H), 3.31 (complex m, 2H), 4.26 (complex m, 2H), 4.83 (d,J=5.6 Hz, 2H), 5.21 ($AB_q$, $J_{AB}$=13.6 Hz, $\Delta\nu_{AB}$=54.3 Hz, 2H), 7.28 (d, J=8.5 Hz, 3H), 7.40 (dd, J=8.6, 1.8 Hz, 1H), 7.49 (d, J=8.4 Hz, 1H), 7.56 (s, 1H), 7.69 (d, J=8.0 Hz, 1H), 7.82 (d, J=1.6 Hz, 1H), 7.91 ppm (d, J=8.7 Hz, 2H);
IR ($CHCl_3$) 3600, 1770, 1720, 1600, 1520 $cm^{-1}$; U.V. ($CH_3CN$): $\lambda_{max}$ 290 nm (ε 10,500), $\lambda_{max}$ 253nm (ε 11,300).

Method 2:

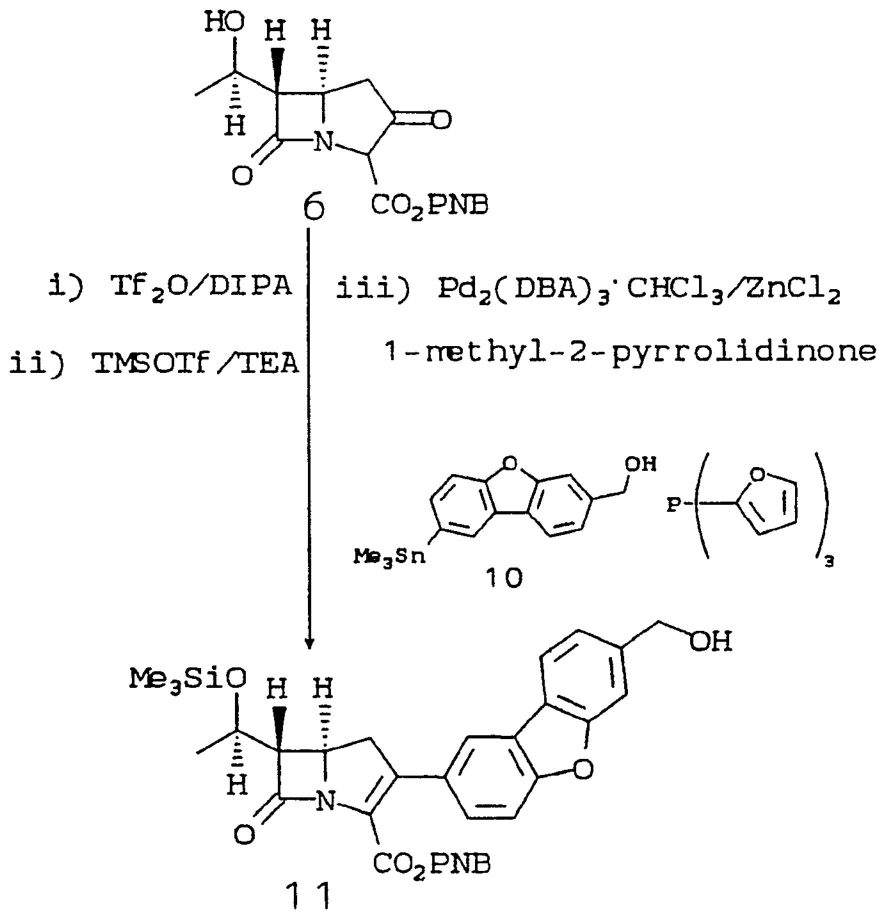


6

10

11

Step A: p-Nitrobenzyl-(5*R*,6*S*) -2-(7-hydroxymethyl-3-dibenzofuranyl)-6-[1*R*-(trimethylsilyloxy)-ethyl]carbapen-2-em-3-carboxylate

A dry 15 mL receiving flask was charged with the bicyclic β-keto carbapenam ester **6** (143 mg; 0.41 mmol) and a magnetic stir bar and the system was purged with nitrogen. Two mL of anhydrous tetrahydrofuran (THF) was added and upon dissolution, the reaction vessel was cooled to -78°C under a nitrogen atmosphere. Diisopropylamine (0.063 mL, 0.45 mmol) was then added and the stirring was continued for 10 minutes.

Trifluoromethanesulfonic anhydride (0.075 mL, 0.45 mmol) was added, followed by stirring for an additional 15 mins. Triethylamine (0.062 mL, 0.45 mmol) was then added, followed by trimethylsilyl trifluoromethanesulfonate (0.087 mL, 0.45 mmol).

While the above reaction was stirred for 20 mins., the organostannane **10** from Step B, Method 1 (178 mg, 0.49 mmol), tris(dibenzylideneacetone)-dipalladium-chloroform (8.5 mg, 0.0082 mmol) and tris(2-furyl)phosphine (3.8 mg, 0.016 mmol) were weighed into a single vial and the vial was purged with nitrogen. When the above reaction time had elapsed, 1-methyl-2-pyrrolidinone (2 mL) was added to the initial reaction mixture followed by the previously weighed solids. A 0.87M zinc chloride in ether solution (1.0 mL, 0.87 mmol) was then added.

The low temperature bath was then removed and the reaction vessel was placed in a 40°C oil bath for 20 min.

The reaction was then quenched by pouring the contents of the flask into a 125 mL separatory funnel containing diethyl ether, ethyl acetate and water. The organic phase was separated and washed with water and brine. The organic phase was dried over magnesium sulfate. The mixture was then filtered and the solvent removed under vacuum. Flash column chromatography of the residue (silica gel, 40% ethyl acetate in hexanes) provided 111 mg of the title carbapenem **11** (47% yield).

Method 3:

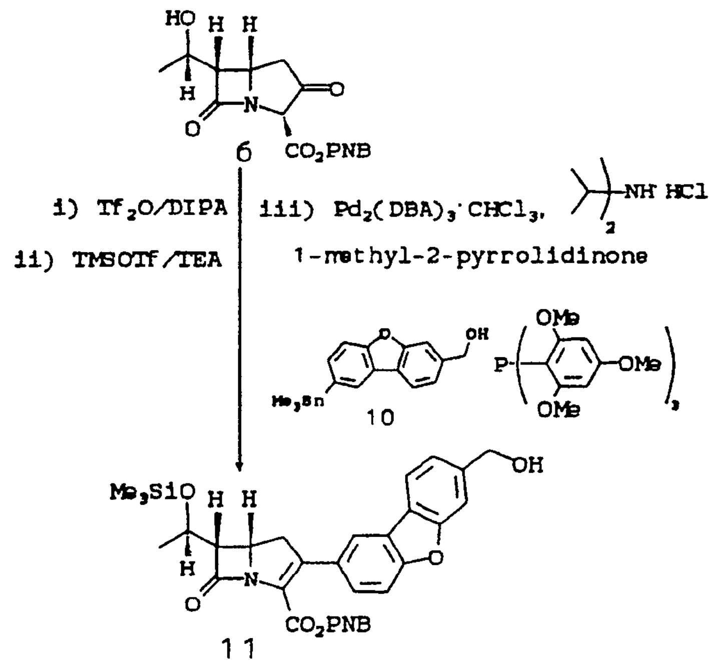


Using the procedure described in Step E of Example 1 but substituting the stannane **10 for the stannane 5** and substituting 1.1 equivalents of diisopropylammonium chloride for 1.1 equivalents zinc chloride in Example 1 provided the title compound "in 48% yield.

EXAMPLE 12

p-Nitrobenzyl-(5***R***,6***S***) -2-(4'-hydroxymethyl-3-biphenyl)-6-[1***R***-(trimethylsilyloxy)ethyl]carbapen-2-em-3-carboxylate

Using the procedure described in Example 9, Method 1 but substituting 3-bromo-4'-(t-butyldimethylsilyloxymethyl)biphenyl for the bromodibenzofuran 8 of Example 9, Method 1 provided the title compound in 67% yield.
$^1$H-NMR (300 MHz, $CDCl_3$): δ 0.14 (s, 9H), 1.29 (d, J=6.1 Hz, 3H), 1.84 (dd, $J_1J_2$=3.0 Hz, 1H), 3.17-3.39 (complex m, 3H), 4.22-4.31 (complex m, 2H), 4.72 (d, J=5.0 Hz, 2H), 5.23 ($AB_q$, $J_{AB}$=13.8 Hz, $\Delta\nu_{AB}$=51.7 Hz, 2H), 7.26-7.55 (complex m, 10H), 8.01 ppm (d, J=8.8 Hz, 2H);
IR ($CHCl_3$) 3600, 1770, 1720, 1600, 1520 cm$^{-1}$;
U.V. ($CH_3CN$):$\lambda_{max}$ 259 nm (ε 11,500).

EXAMPLE 12

p-Nitrobenzyl-(5***R***,6***S***) -2-(4-hydroxymethylphenyl)-6-[1***R***-(trimethylsilyloxy)ethyl]carbapen-2-em-3-carboxylate

Step A: 1-(Trimethylstannyl)-4-(hydroxymethyl)-benzene

Using the procedure described in Steps A and B of Example 9, Method 1 but substituting 1-bromo-4-(t-butyldimethylsilyloxymethyl)benzene for the bromodibenzofuran **8** of Example 9, Method 1 provided the stannyl benzene.
$^1$H-NMR (300 MHz, $CDCl_3$): δ 0.27 (s, 9H), 1.60 (apparent t, J=6.0 Hz, 1H), 4.66 (d, J=6.1 Hz, 2H), 7.33 (d, J=7.6 Hz, 1H), 7.48 ppm (d, J=7.9 Hz, 2H).

Step B: p-Nitrobenzyl-(5*R*,6*S*)-2-(4-hydroxymethyl-phenyl) -6-[1*R*-(trimethylsilyloxy)ethyl]-carbapen-2-em-3-carboxylate

Using the procedure described in Step E of Example 1 but substituting the phenylstannane from Step A for the stannane **5** in Example 1 provided the title compound in 70% yield.
$^1$H-NMR (300 MHz, $CDCl_3$): δ 0.12 (s, 9H), 1.27 (d, J=6.2 Hz, 3H), 3.12-3.32 (complex m, 3H), 4.18-4.29 (complex m, 2H), 4.65 (s, 2H), 5.28 ($AB_q$, $J_{AB}$=14.1 Hz, $\Delta\nu_{AB}$=51.5 Hz, 2H), 7.30 (d, J=1.5 Hz, 4H), 7.44 (d, J=8.7 Hz, 2H), 8.12 ppm (d, J=8.8 Hz, 2H);
IR ($CHCl_3$) 3600, 3540-3300, 1770, 1720, 1600, 1520 $cm^{-1}$; U.V. ($CH_3CN$):$\lambda_{max}$ 270 nm (ε 14,000).

EXAMPLE 13

p-Nitrobenzyl-(5*R*,6*S*) -2-(3-dibenzothienyl)-6-[1*R*-(trimethylsilyloxy)ethyl]carbapen-2-em-3-carboxylate

Step A: 3-(Trimethylstannyl)-dibenzothiophene

Using the procedure described in Step A of Example 9, Method 1 but substituting 3-bromodibenzothiophene for the bromodibenzofuran **8** of Example 9, Method 1 provided the title dibenzothienylstannane in 82% yield.
$^1$H-NMR (300 MHz, $CDCl_3$): δ 0.37 (s, 9H), 7.41-7.48 (complex m, 2H), 7.54 (d, J=8.6 Hz, 1H), 7.82-7.86 (complex m, 2H), 8.18-8.22 (m, 1H), 8.27 ppm (s, 1H).

Step B: P-Nitrobenzyl-(5*R*,6*S*) -2-(3-dibenzothienyl)-6-[1*R*-(trimethylsilyloxy)ethyl]carbapen-2-em-3-carboxylate

Using the procedure described in Step E of Example 1 but substituting the dibenzothienylstannane from Step A for the stannane 5 in Example 1 provided the title compound in 70% yield.
$^1$H-NMR (300 MHz, $CDCl_3$): δ 0.15 (s, 9H), 1.31 (d, J=6.2 Hz, 3H), 3.26-3.45 (complex m, 3H), 4.22-4.35 (complex m, 2H), 5.22 ($AB_q$, $J_{AB}$=13.3 Hz, $\Delta\nu_{AB}$=51.6 Hz, 2H), 7.28 (d, J=8.7 Hz, 2H), 7.37-7.47 (complex m, 3H), 7.58-7.80 (m, 2H), 7.83-7.97 (complex m, 3H), 8.06 ppm (d, J=1.6 Hz, 1H);
IR ($CHCl_3$) 1770, 1720, 1600, 1520 $cm^{-1}$; U.V. ($CH_3CN$): $\lambda_{max}$ 240 nm (ε 14,800).

EXAMPLE 14

p-Nitrobenzyl-(5*R*,6*S*)-2-(9-oxo-3-dibenzothienyl) -6-[1*R*-(trimethylsilyloxy)ethyl]carbapen-2-em-3-carboxylate

Step A: 3-(Trimethylstannyl)-9-oxodibenzothiophene

To a stirred solution of the dibenzothienylstannane from Step A of Example 12 (255 mg, 0.73 mmol) in methylene chloride (7.3 mL) at -78°C under a nitrogen atmosphere was added m-chloroperbenzoic acid (151 mg, 0.88 mmol). The reaction mixture was allowed to warm to 0°C and was stirred at that temperature for 3 hours. The reaction was then quenched with 5% aqueous sodium sulfite. The mixture was then extracted with ether and the organic solution was washed with water and then with saturated aqueous sodium bicarbonate. The organic solution was dried with magnesium sulfate, filtered and concentrated under vacuum. Flash chromatography of the residue (silica gel, 30% EtOAc in hexanes) provided 186 mg of the 9-oxodibenzothienylstannane (70% yield).
$^1$H-NMR (300 Hz, $CDCl_3$): δ 0.36 (s, 9H), 7.44-7.48 (m, 1H), 7.54-7.61 (complex m, 2H), 7.83 (d, J=7.7 Hz, 1H), 7.88-7.97 ppm (complex m, 3H).

Step B: p-Nitrobenzyl-(5*R*,6*S*) -2-(9-oxo-3-dibenzothienyl)-6-[1*R*-(trimethylsilyloxy)ethyl]-carbapen-2-em-3-carboxylate

Using the Procedure described in Step E of Example 1 but substituting the 9-oxodibenzothienyl-stannane from Step A for the stannane **5** in Example 1 provided the title compound in 75% yield.
$^1$H-NMR (300 MHz, $CDCl_3$): δ (diastereomers) 0.14 (s, 9H), 1.28 (d, J=6.2 Hz, 3H), 3.18-3.41 (complex m, 3H), 4.23-4.36 (complex m, 2H), 5.23-5.38 (m, 2H), 7.38-7.65 (complex m, 5H), 7.72 (s, 1H), 7.92-7.98 (m, 2H), 8.06 ppm (dd, J=8.8, 2.2 Hz, 2H);
IR ($CDCl_3$) 1778, 1720, 1600, 1520 $cm^{-1}$; U.V. ($CH_3CN$): $\lambda_{max}$ 250 nm (290+325 shoulder), (ε 27,400).

EXAMPLE 15

p-Nitrobenzyl-(5*R*,6*S*) -2-(2-furyl)-6-[1*R*-(trimethylsilyloxy)ethyl]carbapen-2-em-3-carboxylate

2-(Trimethylstannyl)furan, 69%.
$^1$H-NMR (300 MHz, $CDCl_3$): δ 0.13 (s, 9H), 1.28 (d, J=6.2 Hz, 3H), 3.17 (dd, J=6.2, 2.8 Hz, 1H), 3.24 (dd, J=18.6, 9.1 Hz, 1H), 3.53 (dd, J=8.5, 10.0 Hz, 1H), 4.15-4.27 (complex m, 2H), 5.38 ($AB_q$, $J_{AB}$=14.2 Hz, $\Delta\nu_{AB}$=70.9 Hz, 2H), 6.51 (dd, J=3.5, 1.7 Hz, 1H), 7.45 (d, J=1.7 Hz, 1H), 7.64-7.69 (m, 3H), 8.19 ppm (d, J=8.7 Hz, 2H);
IR ($CHCl_3$) 1770, 1720, 1603, 1520 $cm^{-1}$ ; U.V. ($CH_3CN$): $\lambda_{max}$ 335 nm (ε 16,800), $\lambda_{max}$ 265 nm (ε 12,900).

EXAMPLE 16

p-Nitrobenzyl-(5*R*,6*S*) -2-(2-thienyl)-6-[1*R*-(trimethylsilyloxy)ethyl]carbapen-2-em-3-carboxylate

2-(Trimethylstannyl)thiophene, 41%.
4 Mole % of $Pd_2(DBA)_3 \cdot CHCl_3$ and 16 mole % of tri(2,4,6-trimethoxyphenyl)phosphine were employed.
$^1$H-NMR (300 MHz, $CDCl_3$): δ 0.13 (s, 9H), 1.28 (d, J=6.2 Hz, 3H), 3.19 (dd, J=6.2, 2.7 Hz, 1H), 3.35 (dd, J=17.6, 9.0 Hz, 1H), 3.46 (dd, J=17.6, 9.9 Hz, 1H), 4.16-4.28 (complex m, 2H), 5.39 ($AB_q$, $J_{AB}$=13.9 Hz, $\Delta\nu_{AB}$=67.0 Hz, 2H), 7.06 (dd, J=5.4, 3.9 Hz, 1H), 7.47 (dd, J=4.9, 1.1 Hz, 1H), 7.58 (d, J=2.8 Hz, 1H), 7.67 (d, J=9.2 Hz, 2H), 8.19 ppm (d, J=8.8 Hz, 2H);
IR ($CHCl_3$) 1770, 1710, 1601, 1520 $cm^{-1}$; U.V. ($CH_3CN$): $\lambda_{max}$ 340 nm (ε 12,300), $\lambda_{max}$ 265 nm (ε 13,100).

EXAMPLE 17

p-Nitrobenzyl-(5*R*,6*S*) -2-(2-propenyl)-6-[1*R*-(trimethylsilyloxy)ethyl]carbapen-2-em-3-carboxylate

2-(Trimethylstannyl)propene
$^1$H-NMR (300 MHz, $CDCl_3$): δ 0.11 (s, 9H), 1.25 (d, J=6.1 Hz, 3H), 1.90 (s, 3H), 2.91-3.12 (m, 2H), 3.15 (dd, J=6.2, 2.8 Hz, 1H), 4.10-4.22 (complex m, 2H), 5.03 (s,1H), 5.12 (d, J=1.5 Hz, 1H), 5.32 ($AB_q$, $J_{AB}$=14.0 Hz, $\Delta\nu_{AB}$=46.3 Hz, 2H), 7.61 (d, J=8.7 Hz, 2H), 8.20 ppm (d, J=8.7 Hz, 2H);

EXAMPLE 18

p-Nitrobenzyl-(5*R*,6*S*)-2-(2-Phenylacetylenyl)-6-[1*R*-(t-butyldimethylsilyloxy)ethyl]carbapen-2-em-3-carboxylate

(2-Phenylacetylenyl)trimethylstannane (Note that t-butyldimethylsilyl trifluoromethanesulfonate was substituted for the trimethylsilyl trifluoromethanesulfonate employed in Example 1).
$^1$H-NMR (300 MHz, $CDCl_3$): δ 0.80 (s, 6H), 0.85 (s, 9H), 1.26 (d, J=6.2 Hz, 3H), 3.00-3.19 (m, 2H), 3.23 (dd, J=5.4, 3.6 Hz, 1H), 4.18-4.32 (complex m, 2H), 5.37 ($AB_q$, $J_{AB}$=13.8 Hz, $\Delta\nu_{AB}$=48.0 Hz, 2H), 7.25-7.43 (complex m, 5H), 7.62 (d, J=8.4 Hz, 2H), 8.12 ppm (d., J=8.4 Hz, 2H);

EXEMPLE 19

Potassium (5*R*,6*S*)-2-(1-carbamoyl-3-dibenzofuranyl)-6-[1*R*-hydroxy-ethyl]carbapen-2-em-3-carboxylate

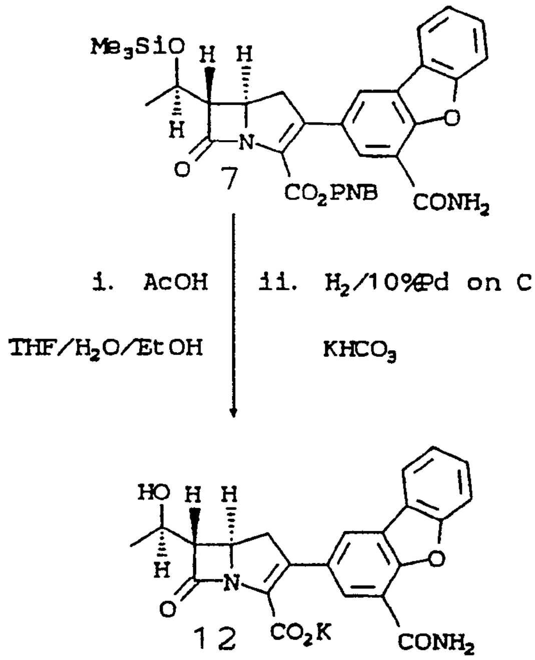


7

12

To a stirred solution of carbapenem 7 from Example 1 (170 mg, 0.277 mmol) in 25 mL of THF/water/EtOH (1.3:1:1.3) was added glacial acetic acid (0.004mL, 0.07 mmol). The solution was heated at 35°C for 70 min., and then potassium bicarbonate (55 mg, 0.55 mmol) was added followed by 10% palladium on carbon (17 mg, 10 wt.%). The reaction vessel was placed under a balloon filled with hydrogen and stirred in this atmosphere for 1 hour at ambient temperature. The reaction mixture was then filtered through a pad of Celite and the pad was rinsed with HPLC grade water. The organic layer was removed under vacuum and the aqueous solution which remained was frozen and lyophilized at 0°C. The residue was purified via reverse-phase thin layer chromatography (4:1 water:acetonitrile) to provide 99 mg of the carbapenem **12** (80.7% yield) as a white solid. This product was identical in all respects to the same compound reported as an example in EP-A-0405774.

EXEMPLE 20

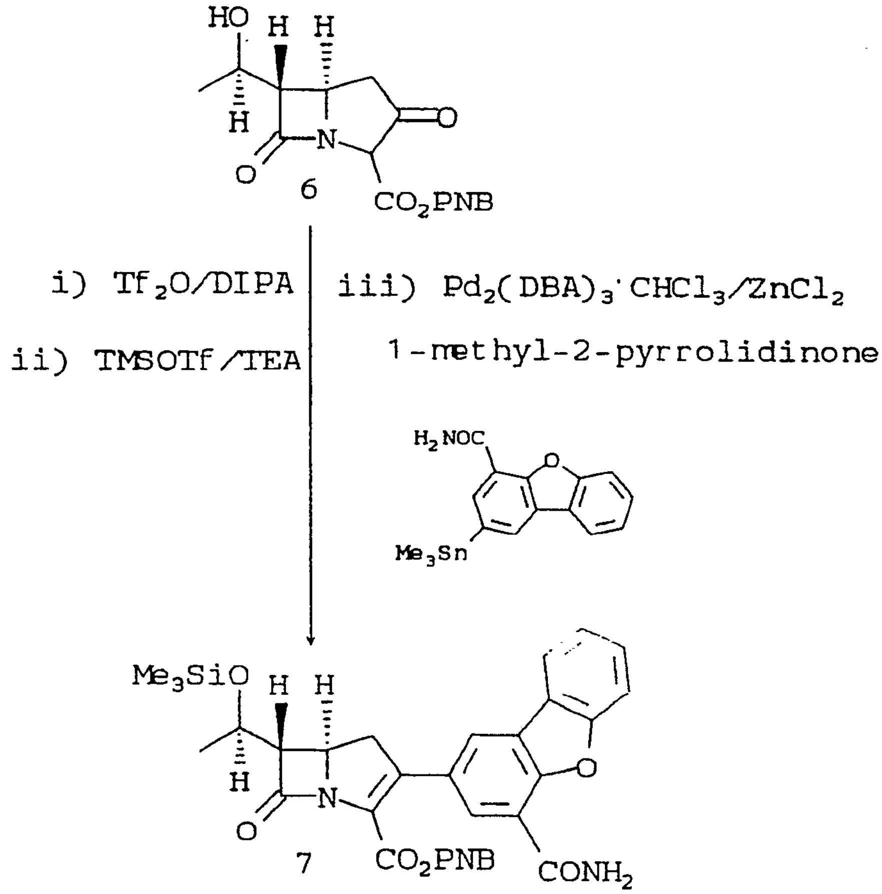


p-Nitrobenzyl-(5***R***,6***S***) -2-(1-carbamoyl-3-dibenzofuranyl)-6-[1***R***-(trimethylsilyloxy)ethyl]carbapen-2-em-carboxylate

A dry 5 mL receiving flask was charged with the bicyclic β-keto carbapenam ester **6** (79.6 mg; 0.228 mmol) and a magnetic stir bar and the system was purged with nitrogen. One mL of anhydrous tetrahydrofuran (THF) was added and upon dissolution, the reaction vessel was cooled to -78°C under $N_2$. Diisopropylamine (0.035 mL, 0.25 mmol) was then added and the stirring was continued for 10 minutes during which time a yellow color developed. Trifluoromethanesulfonic anhydride (0.042 mL, 0.25 mmol) was added, followed by stirring for an additional 15 mins. Triethylamine (0.035 mL, 0.25 mmol) was then added, followed by trimethylsilyltrifluoromethanesulfonate (0.048 mL, 0.25 mmol).

While the above reaction was stirred for 20 mins., the organostannane **5** from Example **1**, Step **D**, (93.8 mg, 0.25 mmol) and tris(dibenzylideneacetone)-dipalladium-chloroform (4.7 mg, 0.0045 mmol, 2 mol %), both solids, were weighed into a single vial and the vial was purged with nitrogen. When the above reaction time had elapsed, dry N-methylpyrrolidinone (1 mL) was added to the initial reaction mixture followed by the previously weighed solids (delivered in one portion). A 1.5M zinc chloride in ether solution (0.167 mL, 0.25 mmol) was then added.

The low temperature bath was then removed and the reaction vessel was placed in a luke warm water bath to allow it to quickly reach ambient temperature. After reaching ambient temperature, the mixture was stirred for 20 minutes during which time a wine red color developed.

The reaction was then quenched by pouring the contents of the flask into a 125 mL separatory funnel containing diethyl ether and water. The organic phase was separated and washed with water (3x) and brine. The organic phase was dried over magnesium sulfate. The mixture was then filtered and the solvent removed under vacuum. Flash column chromatography of the residue (silica gel, 60-65% ethyl acetate/hexanes) provided 108 mg (77%) of carbapenem **7** as a slightly yellowish foam. $^1$H-NMR (300 MHz, $CDCl_3$): δ 0.15 (s, 9H), 1.30 (d, J=6.2 Hz, 3H), 3.28 (dd, J=6.4, 2.7 Hz,1H), 3.31-3.45 (m, 2H), 4.21-4.35 (complex m, 2H), 5.21 ($AB_q$, $J_{AB}$=13.5 Hz, $\Delta\nu_{AB}$=50.1 Hz, 2H), 6.17 (broad singlet, 2H), 7.35-7.41 (m, 3H), 7.48-7.54 (m, 1H), 7.60 (d, J=8.3 Hz, 1H),

7.83 (d, J=7.2 Hz, 1H), 7.97 (d, J=8.8 Hz, 2H), 8.09 (d, J=2.0 Hz, 1H), 8.18 ppm (d, J=1.9 Hz, 1H);
IR ($CHCl_3$): 3510, 3400, 1770, 1720, 1675, 1590, 1520 $cm^1$; U.V. ($CH_3CN$): $\lambda_{max}$ 290 nm (ε 11,000); $\lambda_{max}$ 250nm (ε 13,300)

The following Examples were performed on the same scale and under the conditions as described for Example 19, except as otherwise noted. Each of the following Examples will list the organostannane which was substituted for stannane **5** in Example 19, the yield of the reaction and the pertinent physical and spectroscopic data. If the synthesis of the organostannane employed is not known in the art that synthesis is included in the Example or the synthesis described in Example 10, Method 1, Steps A and B, may be employed but substituting appropriate aryl halides known in the art for the dibenzofuran **8** of Example 10.

EXAMPLE 21

54-Nitrobenzyl-(5*R*,6*S*) -2-(9-oxo-3-dibenzothienyl)-6-[1*R*-(trimethylsilyloxy)ethyl]carbapen-2-em-3-carboxylate

3-(Trimethylstannyl)-9-oxodibenzothiophene (from Example 13, part A) 51%.
Spectral data in accord with that of Example 13, Step B.

EXAMPLE 22

p-Nitrobenzyl-(5*R*,6*S*)-2-(9-hydroxymethyl-3-phenanthrenyl) -6-[1*R*-(trimethylsilyloxy)ethyl]carbapen-2-em-3-carboxylate

3-(Trimethylstannyl)-9-hydroxymethylphenanthrene 71%.
$^1$H-NMR (300 MHz, $CDCl_3$): δ 0.16 (s, 9H), 1.31 (d, J=6.1 Hz, 3H), 2.13 (broad s. 1H), 3.29 (dd, J=6.6, 2.0 Hz,1H), 3.33-3.48 (m, 2H), 4.21-4.38 (complex m, 2H), 5.15 ($AB_q$, $J_{AB}$=13.6 Hz, $\Delta\nu_{AB}$=53.7 H:, 2H), 5.17 (s, 2H), 7.08 (d, J=8.6 Hz, 2H), 7.45 (dd, J=8.2, 1.6 Hz, 1H), 7.56-7.77 (complex m, 6H), 8.05-8.10 (m, 1H), 8.43-8.47 (m, 1H), 8.52 ppm (s, 1H);
IR ($CHCl_3$): 3600, 3520-3350, 1770, 1720, 1600, 1515 $cm^{-1}$; U.V. ($CH_3CN$): $\lambda_{max}$ 252 nm (ε 25,200).

EXAMPLE 23

P-Nitrobenzyl-(5*R*,6*S*)-2-(7-hydroxymethyl-3-dibenzofuranyl) -6-[1*R*-(trimethylsilyloxy)ethyl]carbapen-2-em-3-carboxylate

3-(Trimethylstannyl)-7-hydroxymethyldibenzofuran 66%.
All spectra were in accord with those of the product of Example 10.

EXAMPLE 24

p-Nitrobenzyl-(5*R*,6*S*)-2-(1-[N-carbamoyl)methyl]carbamoyl-3-dibenzofuranyl) -6-[1*R*-(trimethylsilyloxy)-ethyl]carbapen-2-em-3-carboxylate

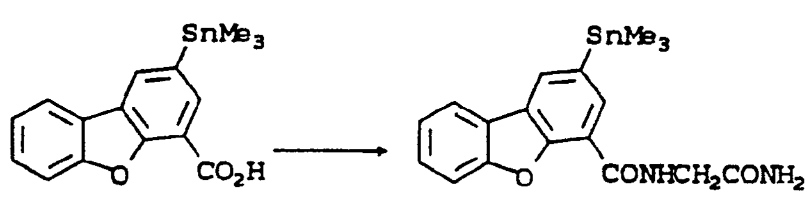

4 13

Step A: 1-[N-Carbamoyl)methyl]carbamoyl-3-(trimethylstannyl)dibenzofuran

To a stirred solution of the stannyl-acid **4** (500 mg, 1.3 mmol) in dry THF(7.5 mL) under $N_2$ was added 1-(3-dimethylaminopropyl)-3-ethylcarbodiimide hydrochloride(307 mg, 1.6 mmol, 1.2 eq) and 1-hydroxybenzotriazole hydrate (270 mg, 2.0 mmol, 1.5 eq). Anhydrous acetonitrile was added to solubilize the resulting

suspension and the mixture was stirred for 30 minutes. A solution of glycinamide hydrochloride (298 mg, 2.6 mmol, 2.0 eq), triethylamine (0.46 mL, 3.3 mmol, 2.5 eq) and DBU (0.2 mL, 1.3 mmol, 1.0 eq) in DMF (10 mL) was then added. After 20 minutes had elapsed, the reaction mixture was poured into EtOAc (200 mL) and washed with water (4 x 25 mL) and brine (2 x 25 mL), then dried ($MgSO_4$), filtered, and concentrated in vacuo. Purification by silica gel flash column chromatography (EtOAc) provided 550 mg (96%) of **13** as a white solid.
$^1$H-NMR (300 MHz, $CDCl_3$): δ 0.38 (s, 9H), 4.31 (d, J=5.6 Hz, 2H), 5.47 (broad s, 1H), 6.27 (broad s, 1H), 7.40 (t, J=7.9 Hz, 1H), 7.50 (t, J=7.8 Hz, 1H), 7.66 (d, J=8.2 Hz, 1H), 8.00 (d, J=7.6 Hz, 1H), 8.20 (s, 1H), 8.27-8.32 ppm (m, 2H).
IR ($CHCl_3$): 3480, 3430, 3000, 1690 cm$^{-1}$.

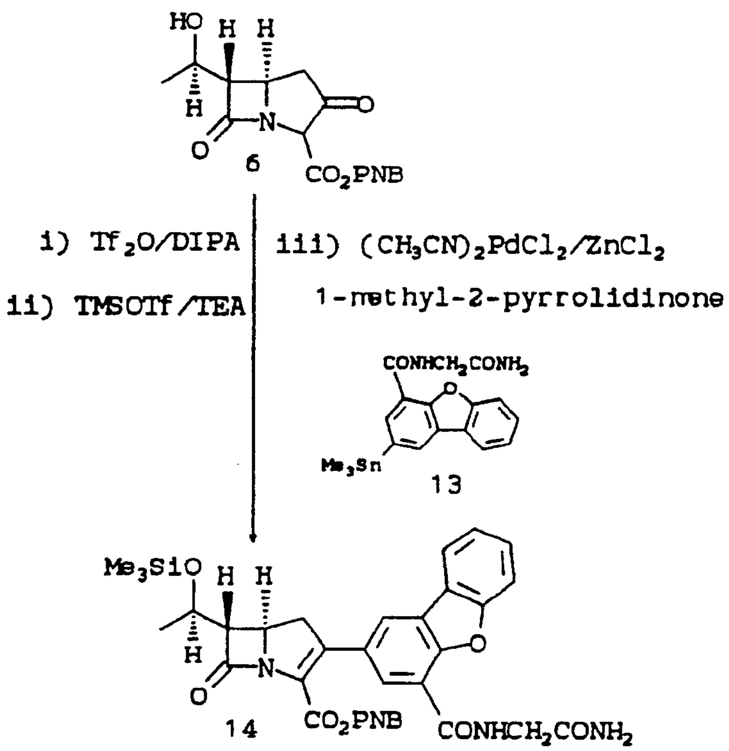


6

13

14

Step B: p-Nitrobenzyl-(5*R*,6*S*)-2-(1-[N-carbamoyl)methyl]carbamoyl-3-dibenzofuranyl) -6-[1*R*-(trimethylsilyloxy)ethyl]carbapen-2-em-3-carboxylate **14**

To a stirred solution of the bicyclic β-keto ester **6** (110 mg, 0.32 mmol) in dry THF (1.6 mL) at -78°C under $N_2$ was added diisopropylamine (53.3 μL, 0.38 mmol, 1.1 eq), and the resultant yellow mixture was stirred for 10 minutes. Trifluoromethanesulfonic anhydride (63.9μL, 0.38 mmol, 1.1 eg) was added and the reaction solution was then stirred for 15 minutes. Triethylamine (53.0 μL, 0.38 mmol, 1.1 eq) and trimethylsilyl trifluoromethanesulfonate (73.4 μL, 0.38 mmol, 1.1 eq) were added and the mixture was stirred for 20 minutes.

Anhydrous N-methyl-2-pyrolidinone (1.6 mL) was added next, followed by addition of the bis-(acetonitrile)palladium (II) chloride catalyst (4.1 mg, 0.16 mmol, 5 mol %) and the aryl stannane **13** from Step A (125mg, 0.29 mmol, 0.91). Finally a 0.87M $ZnCl_2$ in ether solution (0.44 mL, 0.38 mmol, 1.1 eq) was added. The low temperature bath was removed, and the reaction vessel was placed in a warm water bath to quickly reach ambient temperature. The black mixture was then stirred for 30 minutes.

The reaction was quenched by pouring the mixture into a separatory funnel containing ether (40 mL) and water (10 mL). The organic layer was washed with water (3x10 mL) and brine (2 x 10 mL), dried ($MgSO_4$), decolorized briefly with Norite, filtered, and concentrated in vacuo. Purification by silica gel flash chromatography (EtOAc) provided 126 mg (65%) of **14**.
$^1$H-NMR (300 MHz, $CDCl_3$): δ 0.15 (s, 9H), 1.30 (d, J=6.2 Hz, 3H), 3.28 (dd, J=6.4, 2.7 Hz,1H), 3.33-3.38 (m, 2H), 4.24-4.34 (m, 4H), 5.58 (broad s, 1H), 6.22 (broad s, 1H), 7.36-7.43 (m, 3H), 7.52 (t, J=7.2 Hz, 1H), 7.74 (d, J=8.2 Hz, 1H), 7.83 (d, J=7.7 Hz, 1H), 8.01 (d, J=8.8 Hz, 2H), 8.07 (d, J=1.7 Hz, 1H), 8.17 (d, J=1.9 Hz, 1H), 8.27 ppm (m, 1H).

EXAMPLE 25

p-Nitrobenzyl-(5*R*,6*S*)-2-(7-hydroxymethyl-3-dibenzofuranyl) -6-[1*R*-(trimethylsilyloxy)ethyl]carbapen-2-em-3-carboxylate

The title compound was prepared according to the conditions described in Step B of Example 23, but using 3-(trimethylstannyl)-7-hydroxymethyl-dibenzofuran in place of the stannane from Step A Example 23. The final reaction solution was stirred overnight before it was worked-up as previously described to provide the title compound in 44% yield. All spectra were in accord with the product described in Example 9.

EXAMPLE 26

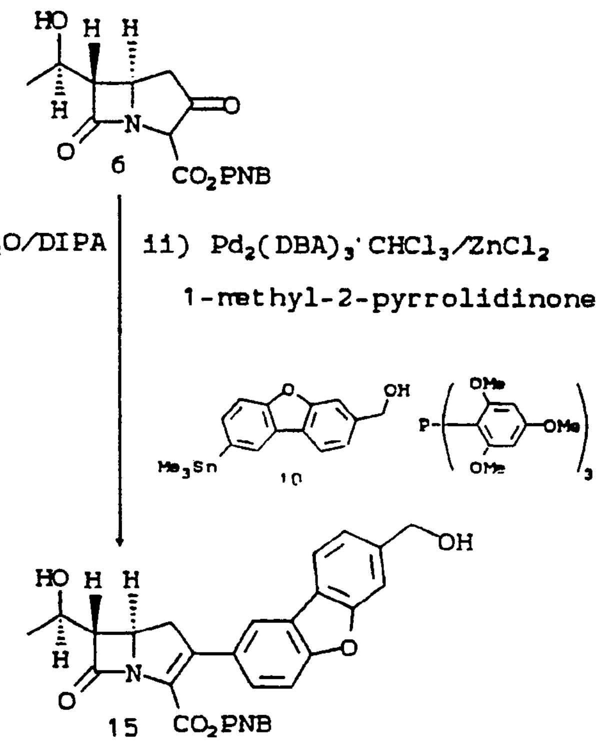


6

10

15

p-Nitrobenzyl-(5*R*,6*S*)-2-(7-hydroxymethyl-3-dibenzofuranyl) -6-[1*R*-(trimethylsilyloxy)ethyl]-carbapen-2-em-3-carboxylate **15**

Method 1:

To a stirred solution of the bicyclic β-keto ester **6** (143 mg, 0.41 mmol) in dry THF (2.0 mL) at -78°C under $N_2$ was added diisopropylamine (63 μL, 0.45 mmol, 1.1 eq), and the resultant yellow mixture was stirred for 10 minutes. Trifluoromethanesulfonic anhydride (75 μL, 0.45 mmol, 1.1 eq) was added and the reaction solution was then stirred for 15 minutes. Anhydrous N-methyl-2-pyrolidinone (2.0 mL) was added next, followed by addition of the tris(dibenzylideneacetone)dipalladium-chloroform (8.5 mg, 2 mol %), tris(2,4,6-trimethoxyphenyl)phosphine (17.4 mg, 0.033 mmol) and the aryl stannane **10** from Example 10, Step B (180mg, 0.45 mmol, 1.1 equiv) in one portion as solids. Finally a 1.5M $ZnCl_2$ in ether solution (0.30 mL, 0.45 mmol, 1.1 eq) was added. The low temperature bath was removed, and the reaction vessel was placed in a warm water bath to quickly reach ambient temperature during which time an intense wine color developed. The reaction was quenched by pouring the mixture into a separatory funnel containing ether (40 mL) and water (10 mL). The organic layer was washed with water and brine, dried ($MgSO_4$), filtered, and concentrated in vacuo. Purification by silica gel flash chromatography (85% EtOAc/hexanes) provided 147 mg (68%) of **15**.

$^1$H-NMR (300 MHz, $CDCl_3$): δ 1.39 (d, J=6.3 Hz, 3H), 1.82 (d, J=4.7 Hz, 1H), 1.95 (t, J=6.0 Hz, 1H), 3.25-3.45 (complex m, 3H), 4.25-4.41 (complex m, 2H), 4.84 (d, J=5.7 Hz, 2H), 5.20 ($AB_q$, $J_{AB}$=13.6 Hz, $\Delta\nu_{AB}$=55.9 Hz, 2H), 7.21-7.31 (complex m, 3H), 7.38 (dd, J=10.3, 1.3 Hz, 1H), 7.49 (d, J=8.3 Hz, 1H), 7.57 (s, 1H), 7.67 (d, J=8.1 Hz, 1H), 7.80 (s, 1H), 7.88 ppm (d, J=8.8 Hz, 2H).

IR ($CHCl_3$): 3600, 3580-3400, 1770, 1720, 1600, 1520 $cm^{-1}$; U.V. ($CH_3CN$): $\lambda_{max}$ 290 nm (ε 22,500), $\lambda_{max}$ 253nm

(ε 24,300).

Method 2:

The title compound **15** was prepared in 83% yield according to the conditions described in Example 25, method 1, but substituting 1.1 equivalents of diisopropylammonium chloride for 1.1 equivalents zinc chloride. All spectra were in accord with the product described in Example 25.

EXAMPLE 27

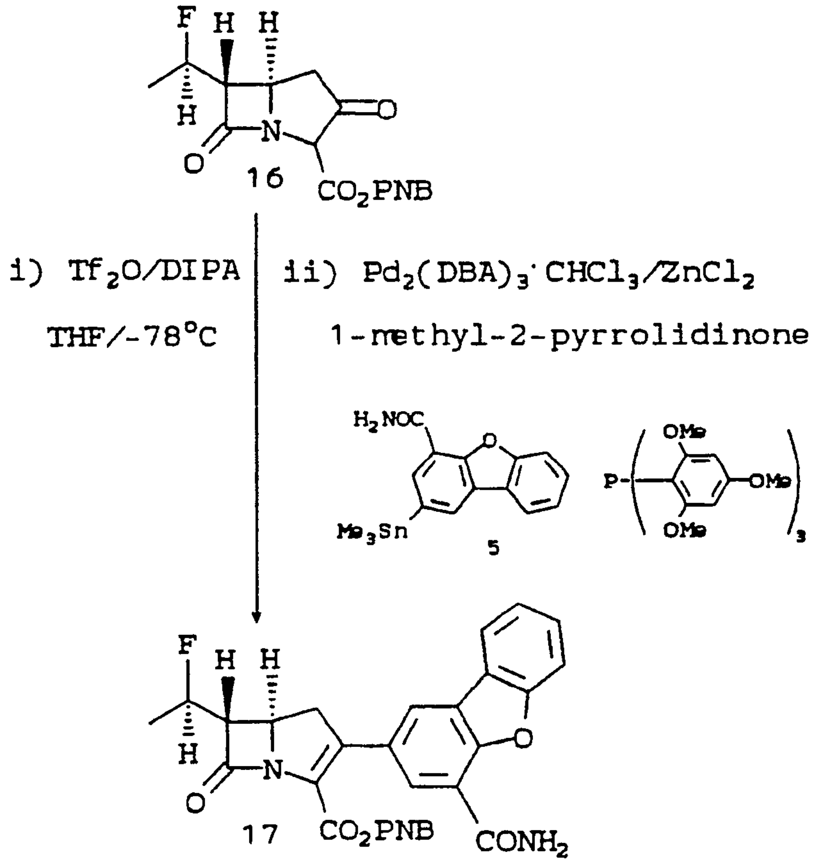


16

5

17

p-Nitrobenzyl-(5*R*,6*R*)-2-(1-carbamoyl-3-dibenzofuranyl)-6-[1*R*-fluoroethyl]carbapen-2-em-3-carboxylate **17**

To a stirred solution of the bicyclic β-keto ester **16** (71 mg, 0.203 mmol) in dry THF (1.0 mL) at -78°C under $N_2$ was added diisopropylamine (31 μL, 0.233 mmol, 1.1 eq), and the resultant yellow mixture was stirred for 10 minutes. Trifluoromethanesulfonic anhydride (38 μL, 0.233 mmol, 1.1 eq) was added and the reaction solution was then stirred for 25 minutes. Anhydrous N-methyl-2-pyrolidinone (1.0 mL) was added next, followed by addition of the tris(dibenzylidene- acetone)dipalladium-chloroform (4.2 mg, 2 mol %), tris(2,4,6-trimethoxyphenyl)-phosphine (8.6 mg, 8 mol %) and the aryl stannane **5** from Example 1, Step D (76mg, 0.233 mmol, 1.1 equiv) in one portion all as solids. Finally a 1.5M $ZnCl_2$ in ether solution (0.135 mL, 0.233 mmol, 1.1 eq) was added. The low temperature bath was removed, and the reaction vessel was placed in a warm water bath to quickly reach ambient temperature during which time an intense wine color developed. The reaction was quenched by pouring the mixture into a separatory funnel containing ether and water. The organic layer was washed with water and brine, dried ($MgSO_4$), filtered, and concentrated in vacuo. Purification by silica gel flash chromatography (50-70% EtOAc/hexanes) provided 89 mg (80%) of **17**.

$^1$H-NMR (300 MHz, $CDCl_3$): δ 1.53 (dd, J=24.0, 6.3 Hz, 3H), 3.32-3.52 (complex m, 3H), 4.39 (dt, J=9.5, 2.6 Hz, 1H), 4.92-5.17 (complex m, 2H), 5.29 (d, J=13.3 Hz, 1H), 6.15-6.25 (broad s, 1H), 7.28-7.42 (complex m, 3H), 7.47-7.64 (complex m, 3H), 7.82 (d, J=7.1 Hz, 1H), 7.95 (d, J=8.7 Hz, 2H), 8.06 (d, J=1.7 Hz, 1H), 8.18 ppm (d, J=1.8 Hz, 1H).

IR ($CHCl_3$) 3500, 3400, 3000, 1780, 1675, 1590, 1520 $cm^{-1}$; U.V. ($CH_3CN$): $\lambda_{max}$ 290 nm (ε 1,700), $\lambda_{max}$ 250nm (ε 2,000).

EXAMPLE 28

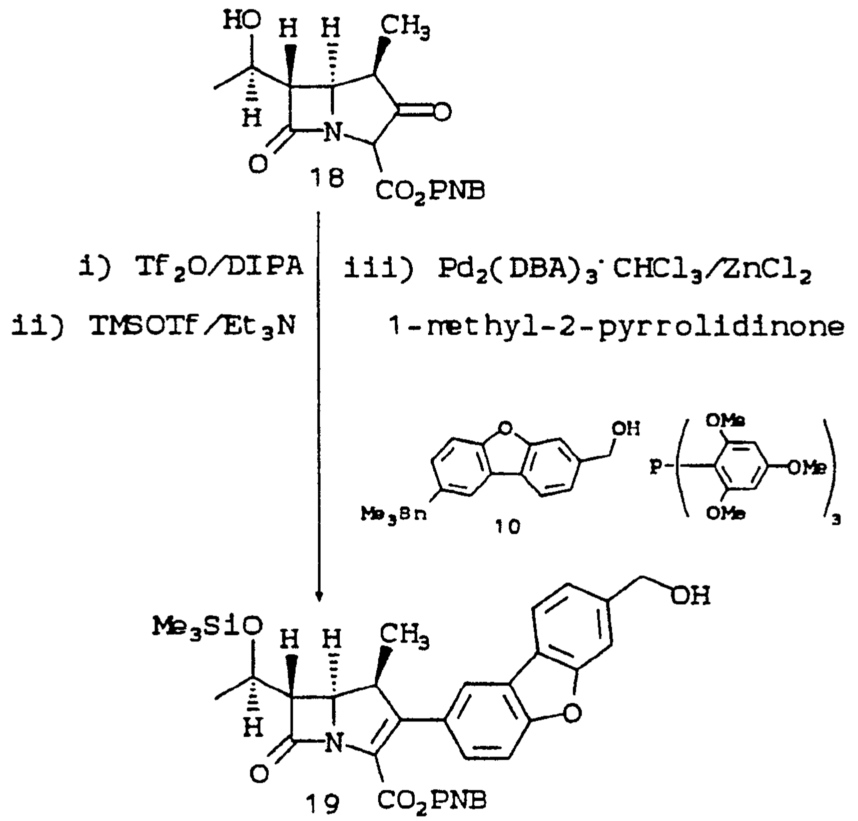


p-Nitrobenzyl-(5*R*,6*S*)-1S-methyl-2-(1-[N-carbamoyl)-methyl]carbamoyl-3-dibenzofuranyl) -6-[1*R*-(trimethylsilyloxy)ethyl]carbapen-2-em-3-carboxylate **19**

To a stirred solution of the bicyclic β-keto ester **18** (38.7 mg, 0.107 mmol) in dry THF (0.5 mL) at -78°C under $N_2$ was added diisopropylamine (16.6 μL, 0.118 mmol, 1.1 eq), and the resultant yellow mixture was stirred for 10 minutes. Trifluoromethanesulfonic anhydride (20.0μL, 0.118 mmol, 1.1 eq) was added and the reaction solution was then stirred for 15 minutes. Triethylamine (16.6 μL, 0.118 mmol, 1.1 eq) and trimethylsilyl trifluoromethanesulfonate (23.0 μL, 0.118 mmol, 1.1 eq) were added and the mixture was stirred for 20 minutes.

While the above reaction was stirred for 20 mins., the organostannane **10** from Example 10, Step B (43 mg, 0.118 mmol), tris(dibenzylideneacetone)-dipalladium-chloroform (2.2 mg, 0.0021 mmol) and tris(2,4,6-trimethoxyphenyl)phosphine (4.6 mg, 0.0086 mmol) were weighed into a single vial and the vial was purged with nitrogen. When the above reaction time had elapsed, N-methylpyrrolidinone (0.5 mL) was added to the initial reaction mixture followed by the previously weighed solids (the solids were added in one portion). A 1.5M zinc chloride in ether solution (0.080 mL, 0.118 mmol) was then added.

The low temperature bath was then removed and the reaction vessel was placed in a luke warm water bath to allow it to quickly reach ambient temperature. After reaching ambient temperature, the mixture was stirred for 1 hour 20 minutes.

The reaction was then quenched by pouring the contents of the flask into a 125 mL separatory funnel containing diethyl ether and water. The organic phase was separated and washed with water (3x) and brine. The organic phase was dried over magnesium sulfate. The mixture was then filtered and the solvent removed under vacuum. Flash column chromatography of the residue (silica gel, 40% ethyl acetate/hexanes) provided 30 mg (45%) of carbapenem **19**.

$^1$H-NMR (300 MHz, $CDCl_3$): δ 0.15 (s, 9H), 1.08 (d, J=15.3 Hz, 3H), 1.29 (d, J=16.1 Hz, 3H), 2.05 (broad s, 1H), 3.36 (dd, J=6.1, 3.1 Hz,1H), 3.48-3.53 (m, 1H), 4.23-4.33 (complex m, 1H), 4.36 (dd, J=10.1, 3.0 Hz, 1H), 5.13 ($AB_q$, $J_{AB}$=13.5 Hz, $\Delta\nu_{AB}$=29.8 Hz, 2H), 7.20 (d, J=8.6 Hz, 2H), 7.28 (d, J=6.8 Hz, 1H), 7.35 (dd, J=8.4, 1.7 Hz, 1H), 7.51 (d, J=8.5 Hz, 1H), 7.57 (s, 1H), 7.69 (d, J=7.9 Hz, 1H), 7.77 (d, J=1.8 Hz, 1H), 7.85 ppm (d, J=8.8 Hz, 1H);

IR ($CHCl_3$) 3600, 3020, 2980, 1770, 1720, 1600, 1520 $cm^{-1}$; U.V. ($CH_3CN$): $\lambda_{max}$ 288 nm (ε 21,400).

## EXAMPLE 29

p-Nitrobenzyl-(5*R*,6*S*)-2-(7-hydroxymethyl-3-dibenzofuranyl)-6-[1R-t-butyldimethylsilyloxy)ethyl]carbapen-2-em-3-carboxylate

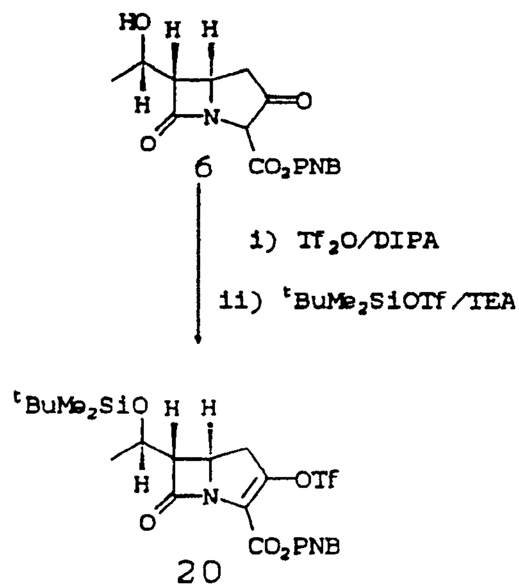


6

20

Step A: p-Nitrobenzyl-(5*R*,6*S*)-2-trifluoromethylsulfonyloxy -6-[1*R*-(t-butyldimethylsilyloxy)-ethyl] carbapen-2-em-3-carboxylate

To the bicyclic β-keto carbapenem ester **6** (106 mg, 0.304 mmol) in 3 mL of anhydrous THF cooled to -78°C under nitrogen was added diisopropylamine (1.1 equivalent, 47 μL, 0.335 mmol). After ten minutes of stirring at this temperature triflic anhydride (1.1 equivalent, 56 μL, 0.335 mmol was added to the resulting yellow solution. An additional fifteen minutes had elapsed before triethylamine (1.1 equivalent, 47 μL, 0.335 mmol) was added followed immediately by tert-butyldimethylsilyl triflate (1.1 equivalent, 77 μL, 0.335 mmol). The reaction mixture was stirred for ten minutes at -78°C before being warmed to ambient temperature for approximately ten minutes. The reaction mixture was then poured into ~40 mL of $Et_2O$ and ~10 mL EtOAc and washed with a saturated solution of $NaHCO_3$ (1x), $H_2O$ (1x), and brine (1x). Drying briefly over $MgSO_4$ was followed by filtration and removal of the solvent in vacuo. Purification by $SiO_2$ flash column chromatography (15% EtOAc/hexanes) provided 161 mg (89%) of **20** as a solid.

$^1H$ NMR (300 MHz , $CDCl_3$) δ: 0.51 (d, J=2.3 Hz, 6H), 0.84 (s, 9H), 1.21 (d, J= 6.3 Hz, 3H), 3.14 (d, J=8.1 Hz, 2H), 3.31 (m, 1H) 4.20-4.31 (complex m, 2H) 5.37 (ABq, $J_{AB}$=13.6 Hz, $\Delta\nu_{AB}$=34.2 Hz, 2H), 7.59 (d, J=8.7Hz , 2H), 8.19 ppm (d, J=8.8 Hz, 2H);

IR ($CHCl_3$): 1800 (s), 1740(s), 1610(m), 1530(s).

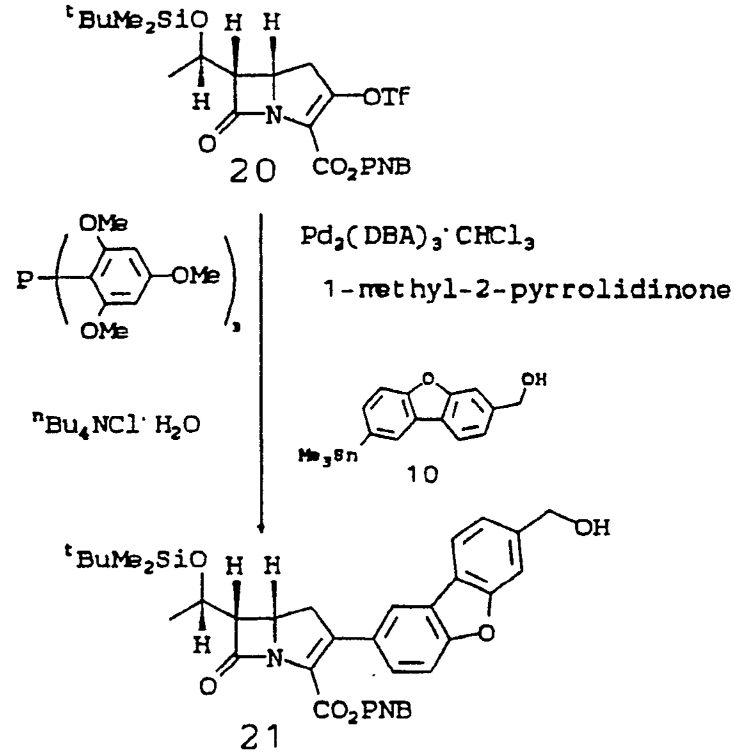


20

10

21

Step B: p-Nitrobenzyl-(5*R*,6*S*)-2-(7-hydroxymethyl-3-dibenzofuranyl) -6-[1*R*-t-butyldimethylsilyloxy) ethyl]carbapen2-em-3-carboxylate

Enol triflate **20** (55.7 mg, 0.094 mmol) was dissolved in a 1.1 mixture of anhydrous THF and N-methylpyrolidinone (1mL) under nitrogen at ambient temperature. To this solution was added as solids all in one portion $Pd_2(DBA)_3 \cdot CHCl_3$ (2 mol %, 2 mg), tris(2,4,6-trimethoxyphenyl)phosphine (8 mol %, 4 mg), stannane **10** (1.1 equivalent; 0.103 mmol, 37 mg), and $H_2O \cdot Bu_4NCl$ (1.1 equivalent, 0.103 mmol, 29 mg). The mixture was sonicated briefly to afford dissolution. After approximately 20 minutes, the resulting yellow solution was poured in $Et_2O$ and washed with water and brine, dried over $MgSO_4$, filtered and solvent removed in vacuo. Purification by $SiO_2$ flash column chromatography (40% EtOAc/hexanes) provided 41.6 mg (69%) of **21**.
$^1H$ NMR (400 MHz, $CDCl_3$) δ 0.09 (d, J=1.5 Hz, 6H), 0.88 (s, 9H), 1.28 (d, J=6.2 Hz, 3H), 3.24-3.40 (complex m, 2H), 4.26-4.36 (complex m, 2H) 4.83 (s, 2H), 5.20 (ABq, $J_{AB}$ = 13.6 Hz, $\Delta\nu_{AB}$ = 69.9 Hz), 7.27-7.31 (complex m, 2H), 7.41 (dd, J = 8.7, 1.8 Hz, 1H), 7.49 (d, J=8.4 Hz, 1H) 7.56 (s, 1H), 7.70 (d, J = 8.0 Hz, 1H), 7.84 (d, J = 1.8 Hz, 1H), 7.92 ppm (d, J = 8.8 Hz, 2H).

EXAMPLE 30

p-Nitrobenzyl-(5*R*,6*S*)-2-{1-[N-2-(N-methyl-2-pyridinium)ethyl]carbamoyl-3-dibenzofuranyl} -6-[1*R*-(trimethylsilyloxy)ethyl]-carbapen-2-em-3-carboxylate trifluoromethanesulfonate

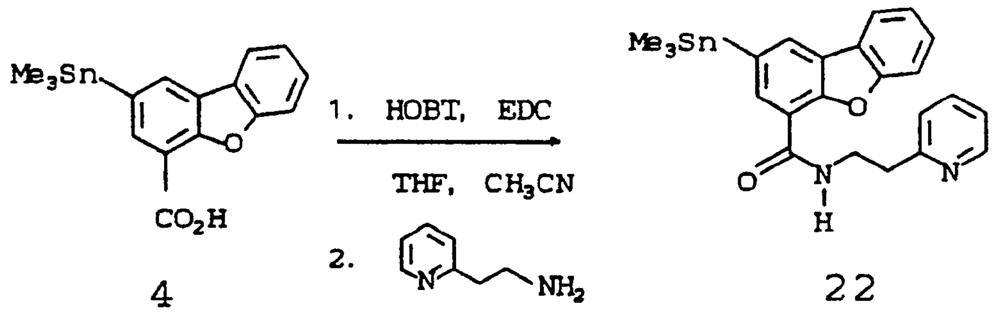


4

22

Step A: 1-[N-2-(2-pyridyl)ethyl]carbamoyl-3-trimethylstannyl-dibenzofuran **(22)**

To a stirred solution of stannyl acid **4** (300 mg, 0.80 mmol) in dry THF (4.5 ml) under $N_2$ was added 1-(3-dimethylaminopropyl)-3-ethylcarbodiimide hydrochloride (183 mg, 0.96 mmol, 1.2 eq.) and 1-hydroxybenzotriazole hydrate (161 mg, 1.2 mmol, 1.5 eq.). Anhydrous $CH_3CN$ was added to solubilize the resulting suspension and the mixture was stirred for 30 minutes. 2-(2-aminoethyl)pyridine (0.14 ml, 1.2 mmol, 1.5 eq.) was then added. After 30 minutes had elapsed, the reaction mixture was poured into ether and washed with $H_2O$ (2x25 ml) and brine (2x25ml), then dried ($MgSO_4$), filtered, and concentrated in vacuo. Purification by silica gel flash column chromatography (70% EtOAc/Hex) provided 375 mg (98%) of 22, as a colorless syrup.
$^1$H-NMR (300 MHz, $CDCl_3$): $\delta$ 0.36 (s, 9H), 3.21 (t, J = 6.6 Hz, 2H,) 4.05 (dd, J = 6.4 Hz, J = 12.6 Hz, 2H) 7.16 - 7.20 (m, 1H), 7.23 (d, J = 7.8 Hz, 1H ), 7.35-7.39 (m, 1H), 7.40-7.51 (m, 2H), 7.59-7.65 (m, 1H), 7.97 (d, J = 7.6 Hz, 1H), 8.14 (s, 1H), 8.26 (s, 1H), 8.33 (s, 1H), 8.64 (d, J = 3.8 Hz, 1H).
IR ($CHCl_3$): 3438, 3065, 3000, 1660 $cm^{-1}$.

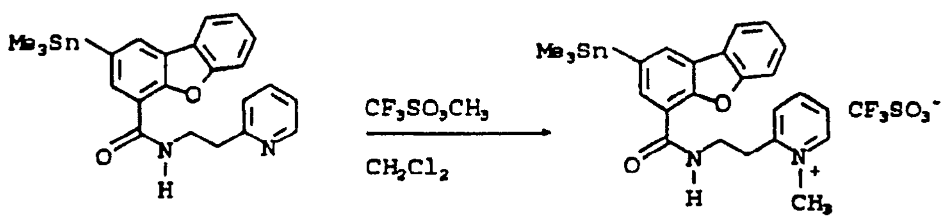


22 23

Step B: 1-[N-2-(N-methyl-2-pyridinium)ethyl]-carbamoyl-3-trimethylstannyl-dibenzofuran trifluoromethanesulfonate (23)

To a stirred solution of 22 (365 mg, 0.76 mmol) in anhydrous $CH_2Cl_2$ (3.8 ml) cooled to 0°C under $N_2$ was added methyl trifluoromethanesulfonate (0.094 ml, 0.83 mmol, 1.1 eq.). The reaction mixture was stirred for 30 minutes at room temperature, then evaporated to provide 412 mg (96%) of **23**.
$^1$H-NMR (300 MHz, $CDCl_3$): $\delta$ 0.34 (s, 9H), 3.51 (t, J = 6.6 Hz, 2H), 3.92-3.99 (m, 2H), 4.44 (s, 3H), 7.27 (t, J = 7.3 Hz, 1H), 7.39 (t, J = 7.3 Hz, 1H), 7.66-7.72(m, 2H), 7.87-7.91 (m, 2H), 8.09-8.14 (m, 3H), 8.30 (apparent t, J = 5.6 Hz, 1H), 8.72 (d, J = 6.5 Hz, 1H).
IR: ($CHCl_3$) 3430, 3060, 3000, 1652 $cm^{-1}$.

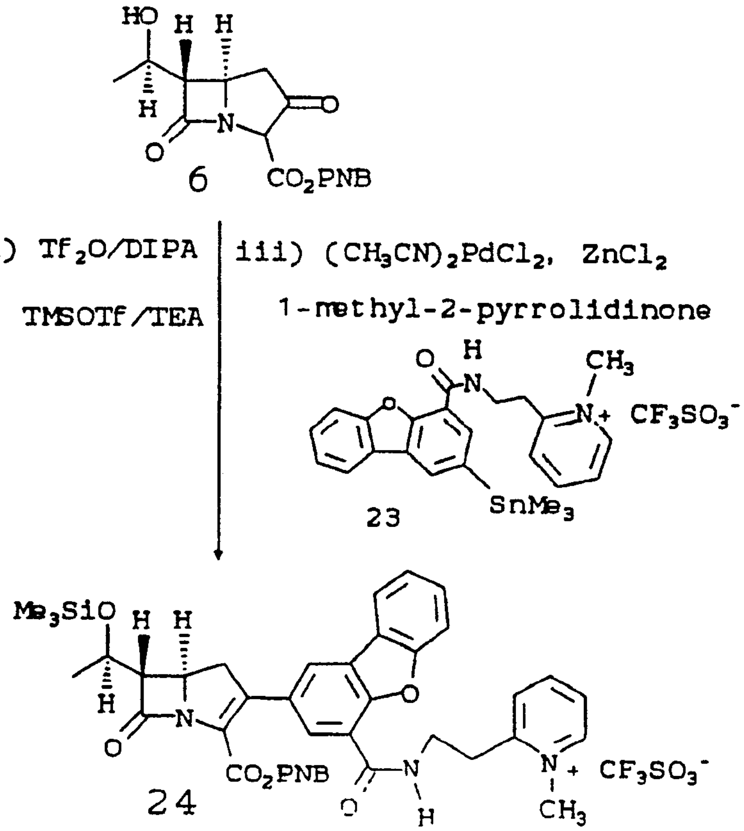


Step C: <u>p-Nitrobenzyl-(5R,6S)-2-{1-[N-2-(N-methyl-2-pyridinium)ethyl]carbamoyl-3-dibenzofuranyl}-6-[1R-(trimethylsilyloxy)ethyl]-carbapen-2-em-3-carboxylate trifluoromethanesulfonate **(24)**</u>

To a stirred solution of the bicyclic β-keto ester **6** (75 mg, 0.12 mmol) in dry THF (0.65 ml) was added diisopropylamine (0.019 ml, 0.14 mmol, 1.1 eq.) at -78°C under $N_2$, and the resultant yellow solution was stirred for 10 minutes. Trifluoromethanesulfonic anhydride (0.023 ml, 0.14 mmol, 1.1 eq.) was added to the reaction mixture, which was then stirred for 15 minutes. Triethylamine (0.019 ml, 0.14 mmol, 1.1 eq.) was added, followed by trimethylsilyl-trifluoromethanesulfonate (0.027 ml, 0.14 mmol, 1.1 eq.) and the solution was stirred for 20 minutes.

Anhydrous N-methyl-2-pyrrolidinone (0.65 ml) was added next, followed by addition of bis(acetonitrile)-palladium(II) chloride (16 mg, $6.3 \times 10^{-3}$ mmol, 5 mol %) and aryl stannane <u>39</u> (75 mg, 0.12 mmol, 0.91 eq.). Finally, a 0.87 <u>M</u> solution of $ZnCl_2$ in ethyl ether (0.16 ml, 0.14 mmol, 1.1 eq) was added. The low temperature bath was removed, and the reaction vessel was placed in a warm water bath to quickly reach ambient temperature. The black mixture was then stirred for 20 minutes.

The reaction was quenched by pouring the mixture into a separatory funnel containing EtOAc (25 ml), $Et_2O$ (10 ml), and $H_20$ (10 ml). The organic phase was then washed with $H_20$ (3x10 ml), dried ($MgSO_4$), decolorized briefly with Norite, filtered, and concentrated <u>in vacuo</u>. Purification by silica gel flash column chromatography (8% $MeOH/CH_2Cl_2$) provided 36 mg (35%) of **24.**

$^1$H-NMR (300 MHz, $CDCl_3$): δ 0.12 (s, 9H), 1.26 (d, J = 6.2 Hz, 3H), 3.27-3.46 (m, 3H), 3.49-3.62 (m, 2H), 3.96-3.99 (m, 2H), 4.24-4.49 (m, 2H), 4.51 (s, 3H), 5.25 (ABq, J = 13.9 Hz, ΔνAB = 48.3 Hz, 2H), 7.34 (t, J = 7.1 Hz, 1H), 7.43-7.51 (m, 3H), 7.73-7.81 (m, 3H), 7.94 (d, J = 7.6 Hz, 1H), 8.00-8.06 (m, 4H), 8.19-8.25 (m, 1H), 8.31-8.35 (m, 1H), 8.64 (d, J = 5.6 Hz, 1H),

IR ($CHCl_3$): 3420, 3000, 2960, 1775, 1725, 1660 $cm^{-1}$. UV ($CH_3CN$): $\lambda_1$ = 319 nm ($\varepsilon_1$ = 10,000), $\lambda_2$= 290 nm ($\varepsilon_2$ = 17,000), $\lambda_3$ = 259 nm ($\varepsilon_3$ = 24,000).

<u>EXAMPLES 31-32</u>

Operating as described in the previous example, the compounds of Table I were analogously prepared.

TABLE I

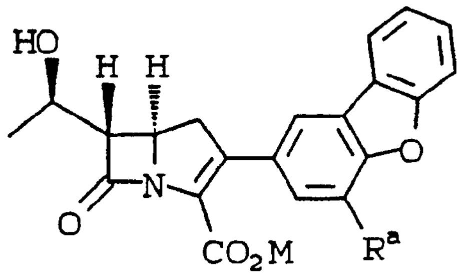

| Example No. | X | M | $\lambda_{max}^{H_2O}$(nm) |
|---|---|---|---|
| 31 | 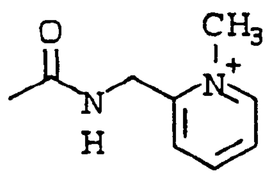 | (-) | 293 |
| 32 | 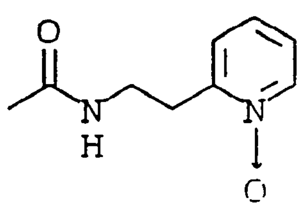 | K | 294 |

**Claims**

1. A Process for preparing a compound of the formula I:

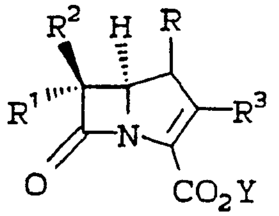

I

wherein:
R is H or $CH_3$;
$R^1$ and $R^2$ are independently H, $CH_3$-, $CH_3CH_2$-, $(CH_3)_2CH$-, $R^4OCH_2$-, $CH_3CH(OR^4)$-, $(CH_3)_2C(OR^4)$—, $FCH_2CH(OR^4)$-, $F_2CHCH(OR^4)$-, $F_3CCH(OR^4)$-, $CH_3CH(F)$-, $CH_3CF_2$-, or $(CH_3)_2C(F)$-;
wherein $R^4$ is hydrogen or $R^5$;
wherein $R^5$ is triorganosilyl, p-nitrobenzyloxycarbonyl or allyloxycarbonyl;
$R^3$ is a mono- or disubstituted or unsubstituted group which is: alkyl, alkenyl, alkynyl, phenyl, furyl, thienyl, dibenzofuranyl, biphenyl, phenanthrenyl, fluoren-9-onyl, pyridyl, phenylpyridyl, naphthyl, dibenzothienyl, 9-oxodibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;
b) a mono- or disubstituted or unsubstituted group which is: alkyl, alkenyl and alkynyl wherein the substituent (or substituents) is selected from a) hereinabove;
Y is allyl, substituted allyl, benzyl, substituted benzyl, alkyl, substituted alkyl or triorganosilyl;
COMPRISING THE STEP OF TREATING the carbapenem compound of the Formula II:

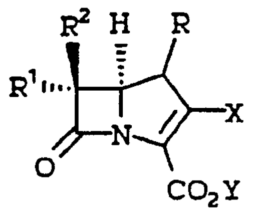

II

having the same substituents R, $R^1$, $R^2$, and Y as described above and
wherein X is trifluoromethanesulfonyloxy, methanesulfonyloxy, p-toluenesulfonyloxy, chloro, bromo, iodo or diphenylphosphonyloxy;
with an organostannane of the Formula III:

$$R^s_3Sn—R^3 \quad III$$

wherein $R^s$ is lower alkyl, and $R^3$ is as described above;
IN THE PRESENCE OF a palladium compound and a halide source in an aprotic polar coordinating solvent and optionally in the presence of a phosphine of Formula IV:

$$PC(R^p)_3 \quad IV$$

wherein $R^p$ is a group substituted from one to three times or unsubstituted which is: phenyl, 2-furyl or 2-thienyl:
wherein the substituent (or substituents) is lower alkyl or lower alkoxy.

2. The process according to Claim 1 wherein $R^p$ is 2,4,6-trimethoxyphenyl, 4-methoxyphenyl or 2,6-dimethoxyphenyl.

3. The process according to Claim 1 wherein the phosphine of Formula IV is not present.

4. The process according to Claim 1 wherein a palladium compound is $Pd_2(DBA)_3·CHCl_3$.

5. The process according to Claim 1 wherein a halide source is zinc chloride or a substituted ammonium halide.

6. The process according to Claim 1 wherein an aprotic coordinating solvent is 1-methyl-2-pyrrolidinone.

7. A process for preparing a compound of the formula I:

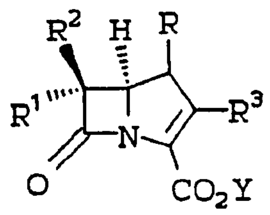

I

wherein:
R is H or $CH_3$;
$R^1$ is (**R**)-$CH_3CH(OR^4)$- or (**R**)-$CH_3CHF$-;
wherein $R^4$ is hydrogen or $R^5$;
wherein $R^5$ is triorganosilyl, p-nitrobenzyloxycarbonyl or allyloxycarbonyl;
$R^2$ is hydrogen;
$R^3$ is a mono- or disubstituted or unsubstituted group which is: alkyl, alkenyl, alkynyl, phenyl, furyl, thienyl, dibenzofuranyl, biphenyl, phenanthrenyl, fluoren-9-onyl, pyridyl, phenylpyridyl, naphthyl, dibenzothienyl, 9-oxodibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;
b) a mono- or disubstituted or unsubstituted group which is alkyl, alkenyl and alkynyl wherein the substituent (or substituents) is selected from a) hereinabove;
Y is allyl, substituted allyl, benzyl, substituted benzyl, alkyl, substituted alkyl or triorganosilyl;
COMPRISING THE STEP OF TREATING the carbapenem compound of the Formula II:

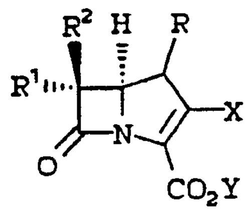

II

having the same substituents R, $R^1$, $R^2$, and Y as described above and wherein X is trifluoromethanesulfonyloxy;
with an organostannane of the Formula III:

$R^s{}_3Sn—R^3$ III

wherein $R^s$ is lower alkyl, and $R^3$ is as described above;
IN THE PRESENCE OF a palladium compound and a halide source in an aprotic polar coordinating solvent and, optionally, in the presence of a phosphine of Formula IV:

$P(R^p)_3$ IV

wherein $R^p$ is 2,4,6-trimethoxyphenyl, 4-methoxyphenyl or 2,6- dimethoxyphenyl.

8. The process according to Claim 7 wherein:

$R^3$ is a mono- or disubstituted or unsubstituted group which is: phenyl, furyl, thienyl, dibenzofuranyl, biphenyl, phenanthrenyl, fluoren-9-onyl, naphthyl, dibenzothienyl, 9-oxo- dibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl,

carbonylamino or amidino, wherein the substituent (or substituents) is alkyl or aryl;
b) a mono- or disubstituted or unsubstituted group which is alkyl, alkenyl and alkynyl wherein the substituent (or substituents) is selected from a) hereinabove.

9. The process according to Claim 7 wherein Palladium compound is $Pd_2(DBA)_3{\cdot}CHCl_3$, halide source is zinc chloride or a substituted ammonium halide, and aprotic coordinating solvent is 1-methyl-2-pyrrolidinone and all of the other variables and substituents are as described in Claim 7.

10. The process of Claim 7 wherein the carbapenem compound of the formula II is produced in situ from the β-keto ester carbapenam of the formula V:

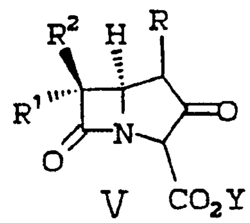

V

wherein
R is H or $CH_3$;

$R^1$ is (*R*)-$CH_3CH(OR^4)$- or (*R*)-$CH_3CHF$-;
wherein R4 is hydrogen or $R^5$;
wherein $R^5$ is triorganosilyl, p-nitrobenzyloxycarbonyl or allyloxycarbonyl;
$R^2$ is hydrogen;
Y is allyl, substituted allyl, benzyl, substituted benzyl, alkyl, substituted alkyl or triorganosilyl.

11. A process for preparing the compound of the formula I:

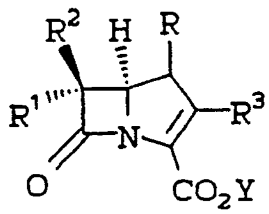

I

wherein:
R is H;
$R^1$ is (*R*)-$CH_3CH(OR^4)$- or (*R*)-$CH_3CHF$-;
wherein $R^4$ is hydrogen or trimethylsilyl;
$R^2$ is hydrogen;
$R^3$ is a mono- or disubstituted or unsubstituted group which is: alkyl, alkenyl, alkynyl, phenyl, furyl, thienyl, dibenzofuranyl, biphenyl, phenanthrenyl, fluoren-9-onyl, pyridyl, phenylpyridyl, naphthyl, dibenzothienyl, 9-oxodibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;
b) a mono- or disubstituted or unsubstituted group which is: alkyl, alkenyl and alkynyl wherein the substituent (or substituents) is selected from a) hereinabove;
Y is 4-nitrobenzyl;
COMPRISING THE STEP OF TREATING the carbapenem compound of the Formula II:

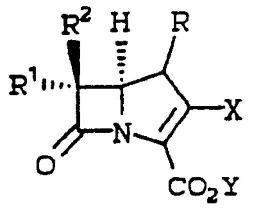

II

having the same substituents R, $R^1$, $R^2$, and Y as described above and wherein X is trifluoromethanesulfonyloxy;

with an organostannane of the Formula III:

$R^s{}_3\,Sn—R^3$ III

wherein $R^s$ is methyl, and $R^3$ is as described above;

IN THE PRESENCE OF a halide source chosen from zinc chloride and a substituted ammonium halide; and in the presence of tris(dibenzylidineacetone)-dipalladium-chloroform in 1:1 tetrahydrofuran: 1-methyl-2-pyrrolidinone.

**12.** The process according to Claim 11 wherein:
$R^3$ is a mono- or disubstituted or unsubstituted group which is: phenyl, furyl, thienyl, dibenzofuranyl, biphenyl, phenanthrenyl, fluoren-9-onyl, naphthyl, dibenzothienyl, 9-oxo- dibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkoxy, mercapto, carbonyl, carbonyloxy, carbamoyl, carbamoyloxy, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;
b) a mono- or disubstituted or unsubstituted group which is alkyl, alkenyl and alkynyl wherein the substituent (or substituents) is selected from a) hereinabove.

**13.** A process of Claim 1 wherein a compound of the formula I is deprotected to provide a carbapenem antibiotic of the formula VI:

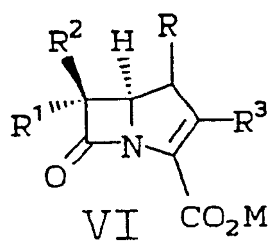

VI

wherein
R is H or $CH_3$;

$R^1$ and $R^2$ are independently H, $CH_3$-, $CH_3CH_2$-, $(CH_3)_2CH$-, $R^4OCH_2$-, $CH_3CH(OR^4)$-, $(CH_3)_2C(OR^4)$—, $FCH_2CH(OR^4)$-, $F_2CHCH(OR^4)$-, $F_3CCH(OR^4)$-, $CH_3CH(F)$-, $CH_3CF_2$-, or $(CH_3)_2C(F)$-;
wherein $R^4$ is hydrogen;
$R^3$ is a mono or disubstituted or unsubstituted group which is: alkyl, alkenyl, alkynyl, phenyl, furyl, thienyl, dibenzofuranyl, biphenyl, phenanthrenyl, fluoren-9-onyl, pyridyl, phenylpyridyl, naphthyl, dibenzothienyl, 9-oxodibenzothienyl or 9,9-dioxodibenzothienyl;
wherein the substituent (or substituents) is:
a) a substituted or unsubstituted group which is: amino, hydroxyl, cyano, carboxyl, nitro, chloro, bromo, fluoro, alkory, mercapto, carbonyl, carbonyloxy, carbamoyl, carbamoylory, thio, sulfonyl, sulfino, thiocarbamoyl, thiocyanate, formyl, phosphonyl, phosphinyl, phosphoramido, azido, ureido, sulfamoyl, carbonylamino or amidino, wherein the substituent (or substituents) is alkyl or aryl;
b) a mono- or disubstituted or unsubstituted group which is: alkyl, alkenyl and alkynyl wherein the substituent (or substituents) is selected from a) hereinabove;
M is hydrogen or alkali metal.

14. A compound of the formula:

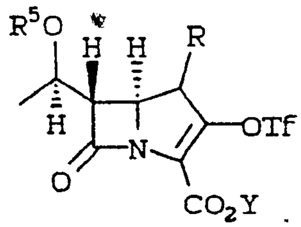

wherein
R is H or $CH_3$;

$R^5$ is triorganosilyl, p-nitrobenzyloxycarbonyl or allyloxycarbonyl;

Y is allyl, substituted allyl, benzyl, substituted benzyl, alkyl, substituted alkyl or triorganosilyl.

European Patent Office

# EUROPEAN SEARCH REPORT

Application Number
EP 91301557.4

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 105, no. 9, Semptember 1, 1986, Columbus, Ohio, USA BUMAGIN N.A. et al. "Palladium- and nickel -catalyzed aryl- and acyl -demetalation of organo metallic compounds" page 653, column 1, abstract-no. 78 973a & J. Organomet. Chem., 1985, 291(1), 129-32 -- | 1,4 | C 07 D 477/00 |
| A | CHEMICAL ABSTRACTS, vol. 105, no. 9, September 1, 1986, Columbus, Ohio, USA SCOTT W.J. et al. "Palladium- catalyzed coupling of vinyl triflates with organostannanes" page 660, column 2, abstract-no. 79 075w & J. Am. Chem. Soc., 1986, 108(11), 3 033-40 -- | 1,4,5 | TECHNICAL FIELDS SEARCHED (Int. Cl.5) C 07 D 477/00 |
| A | CH - A5 - 657 853 (SANKYO) * Claim 20 * ---- | 1,14 | |

The present search report has been drawn up for all claims

| Place of search | Date of completion of the search | Examiner |
|---|---|---|
| VIENNA | 29-04-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)